(19) **Europäisches Patentamt / European Patent Office / Office européen des brevets**

(11) **EP 4 219 681 A1**

(12) # EUROPEAN PATENT APPLICATION
published in accordance with Art. 153(4) EPC

(43) Date of publication:
**02.08.2023 Bulletin 2023/31**

(21) Application number: **21872985.3**

(22) Date of filing: **27.09.2021**

(51) International Patent Classification (IPC):
*C12N 1/20* (2006.01)    *C12Q 1/6883* (2018.01)
*C12Q 1/689* (2018.01)    *G01N 33/68* (2006.01)
*G01N 33/569* (2006.01)    *A61K 35/74* (2015.01)
*A61P 1/00* (2006.01)    *C12R 1/01* (2006.01)

(52) Cooperative Patent Classification (CPC):
**A61K 35/74; A61P 1/00; C12N 1/20; C12Q 1/6883;
C12Q 1/689; G01N 33/569; G01N 33/68;**
C12R 2001/01

(86) International application number:
**PCT/KR2021/013152**

(87) International publication number:
**WO 2022/065957 (31.03.2022 Gazette 2022/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.09.2020 KR 20200126243**

(71) Applicant: **CJ Bioscience, Inc.**
**Jung-gu**
**Seoul 04527 (KR)**

(72) Inventors:
• **HONG, Sang Hi**
  **Seoul 06725 (KR)**
• **LEE, Je Hee**
  **Seoul 04739 (KR)**

• **CHOE, Kyoung-Jin**
  **Pocheon-si, Gyeonggi-do 11147 (KR)**
• **YOON, Seok-Hwan**
  **Seoul 06218 (KR)**
• **OH, Hyeonseok**
  **Seoul 08802 (KR)**
• **KIM, Hyun**
  **Seoul 06725 (KR)**
• **KIM, Dahye**
  **Seoul 06725 (KR)**

(74) Representative: **Bates, Philip Ian**
**Reddie & Grose LLP**
**The White Chapel Building**
**10 Whitechapel High Street**
**London E1 8QS (GB)**

Remarks:
The complete document including Reference
Table(s) and the Sequence Listing(s) can be
downloaded from the EPO website

(54) **COMPOSITION FOR DIAGNOSIS OR TREATMENT OF INFLAMMATORY DISEASES, COMPRISING MICROORGANISM**

(57)    The present invention relates to a Faecalibacterium sp. microorganism and a composition for diagnosis or treatment of bowel diseases, comprising same.

EP 4 219 681 A1

**(Cont. next page)**

【FIG. 8】

**Description**

[TECHNICAL FIELD]

The present invention relates to a composition for diagnosis or treatment of inflammatory disease comprising a *Faecalibacterium* spp. microorganism.

[BACKGROUND ART]

Inflammation occurs when an organism's immune system attempts to defend harmful substances such as invasive pathogens. Immunomodulatory mechanisms, including immunomodulatory cells, cytokines and apoptosis, regulate immune responses caused by an overreaction to defend against pathogens. The deficiency of the mechanisms manifests inflammatory diseases including inflammatory bowel disease (IBD). The inflammatory disease includes for example an inflammatory gastrointestinal disease selected from the group consisting of inflammatory bowel disease (Crohn's disease, ulcerative colitis), irritable bowel syndrome, chronic digestive disorder (coeliac disease), infectious colitis (for example, caused by C. difficile), Behcet's disease, other gastrointestinal-related diseases, and any combination thereof.

Inflammatory bowel disease (IBD) is a chronic inflammatory condition of the gastrointestinal tract, characterized by the disrupted mucosal barrier, altered composition of intestinal microorganism and systemic biochemical abnormality. IBD is categorized into ulcerative colitis (UC) and Crohn's disease (CD) according to clinical features and inflammation localization in intestine. The accurate etiologic cause of IBD is not yet known, but it is known that an excessive immune response triggered by altered intestinal flora or environmental factors of pathogenic microorganisms is a main etiologic cause.

IBD is a disease caused by a complex and unknown etiologic cause, and treatment for IBD is very limited, and focuses on inducing remission and maintaining remission by improving symptoms rather than treatment. An optimal treatment method may be selected in consideration of the regional range of lesions, severity and clinical features, complications and the like.

Therapeutic agents of IBD show an effect through various mechanisms of action, and thus, they are being used step by step according to detailed clinical guidelines, and 5-aminosalicylate and corticosteroid are often used as primary therapy for mild to moderate IBD, and biological agents such as TNF-$\alpha$ inhibitors (e.g., infliximab, adalimumab and golimumab, etc.) are recommended as a secondary medicine for a patient who does not respond or fail to the primary treatment. However, patients who are administered with TNF-$\alpha$ inhibitor at a high dose in the early stage of disease is increasing sharply. The long-term use of a powerful immunosuppressive agent has a problem in serious side-effect, and thus, there is an ongoing clinical need for alternative therapies.

The relationship between IBD and intestinal flora has been confirmed in many studies would wide, and it has been reported that in IBD patients, microbiota dysbiosis characterized by deficiency of beneficial gut bacteria is shown, and the microbiota dysbiosis is being considered as immunopathogenesis of IBD.

[DISCLOSURE]

[TECHNICAL PROBLEM]

An embodiment of the present invention is to provide a composition, kit or method for predicting a risk of developing inflammatory disease, specifically, inflammatory gastrointestinal disease, or diagnosing inflammatory disease, specifically, inflammatory gastrointestinal disease, for example, inflammatory bowel disease using a *Faecalibacterium* spp. Microorganism, or for predicting or evaluating responsiveness of prevention, improvement or treatment to inflammatory disease, specifically, inflammatory gastrointestinal disease, for example, inflammatory bowel disease, using the microorganism.

[TECHNICAL SOLUTION]

An embodiment of the present invention provides a composition for prevention, improvement or treatment of inflammatory disease, specifically, inflammatory gastrointestinal disease, for example, inflammatory bowel disease, comprising at least one selected from the group consisting of; a microbial cell of *a Faecalibacterium* spp.; a culture of the microorganism; a lysate of the microorganism; and at least an extract selected from the group consisting of the microbial cell, culture and lysate. The composition may be a pharmaceutical or food composition.

In addition, the present invention relates to diagnosis of inflammatory disease, specifically, inflammatory gastrointestinal disease, for example, inflammatory bowel disease, including for example, selection of a subject to be administered or evaluation of prevention, improvement or treatment efficacy of inflammatory disease, specifically, inflammatory gastrointestinal disease, for example, inflammatory bowel disease.

An embodiment of the present invention provides a method, composition or kit for diagnosis of bowel disease comprising at least one selected from the group consisting of; a microbial cell of a *Faecalibacterium* spp.; a culture of the microorganism; a lysate of the microorganism; and at least an extract selected from the group consisting of the microbial cell, culture and lysate; a method, composition or kit for selecting a subject to which a microbial cell of a *Faecalibacterium* spp. and the like is administered; or a method, composition or kit for evaluating prevention, improvement or treatment efficacy of inflammatory disease, specifically, inflammatory gastrointestinal disease, for example, inflammatory bowel disease by administering a microbial cell of a *Faecalibacterium* spp. into a subject.

An embodiment of the present invention relates to a composition for prevention, improvement or treatment of inflammatory disease, specifically, inflammatory gastrointestinal disease or inflammatory bowel disease, comprising at least one selected from the group consisting of anti-inflammatory agents, corticosteroids, immunomodulators, antibiotics, tumor necrosis factor inhibitors (tumor necrosis factor-$\alpha$ agent inhibitor, TNF-$\alpha$ inhibitor), Janus kinase inhibitors (e.g., tofacitinib, etc.), anti-integrin agents (e.g., vedolizumab, etc.), interleukin inhibitors (e.g., ustekinumab, etc.), and the like, in addition to at least one selected from the group consisting of; a microbial cell of a *Faecalibacterium* spp.; a culture of the microorganism; a lysate of the microorganism; and at least an extract selected from the group consisting of the microbial cell, culture and lysate. The example of the anti-inflammatory agent includes 5-aminosalicylate, sulfasalazine and olsalazine. The example of the steroids includes corticosteroids, glucocorticosteroids, prednisone, hydrocortisone, methylprednisolone, dexamethasone and adrenocorticotropic hormone (hereinafter, abbreviated as "ACTH".). The example of the immunomodulator includes deglycolipidated, deglycolipidated Mycobacterium vaccae (hereinafter, abbreviated as "PVAC".), anti-tumor necrosis factor receptor superfamily member 5 (hereinafter, abbreviated as "anti-CD40".) ligands, anti-CD40, natalizumab (AntegrenTM), anti-vascular adhesion molecule 1 (hereinafter, abbreviated as "anti-VCAM1".) and anti-intercellular adhesion molecule-1 (hereinafter, abbreviated as "anti-ICAM1".). The example of the cytokine includes interleukin-10 (hereinafter, abbreviated as "IL-10".). The example of the TNF antagonist includes infliximab (Remicade0), etanercept (EnbrelOO), adalimumab (Humira), and Certolizumab Pegol (hereinafter, abbreviated as "CDP870".). An example of the Janus kinase inhibitor includes Tofacitinib (Xeljanz), and an example of the anti-integrin agent includes vedolizumab (KyntelesR), and an example of the interleukin inhibitor includes ustekinumab (Stelara pfs).

An embodiment of the present invention provides a composition, kit or method for selecting a subject to be administered with a *Faecalibacterium* spp. Specifically, the present invention predicts a risk of developing inflammatory gastrointestinal disease, for example, inflammatory bowel disease, or diagnoses gastrointestinal disease, for example, inflammatory bowel disease, or predicts or evaluates responsiveness of prevention, improvement or treatment to inflammatory gastrointestinal disease, for example, inflammatory bowel disease according to administration of the microorganism, by detecting *Faecalibacterium cancerinhibens* in a sample of a test subject, and accordingly, it may determine whether the subject can receive improvement or treatment therapy of gastrointestinal disease, for example, inflammatory bowel disease.

An embodiment of the present invention provides a composition, kit or method for evaluating efficacy or responsiveness of *Faecalibacterium cancerinhibens* in a subject administered with *Faecalibacterium cancerinhibens,* using a *Faecalibacterium* spp. Specifically, an embodiment of the present invention relates to a composition or kit for predicting a risk of developing gastrointestinal disease, for example, inflammatory bowel disease, or diagnosing gastrointestinal disease, for example, inflammatory bowel disease, or predicting or evaluating responsiveness of prevention, improvement or treatment to gastrointestinal disease, for example, inflammatory bowel disease, comprising an agent for detecting *Faecalibacterium cancerinhibens* in a biological sample of a test subject.

An additional embodiment of the present invention relates to a method for predicting a risk of developing inflammatory disease, specifically, inflammatory gastrointestinal disease, for example, inflammatory bowel disease, or diagnosing inflammatory disease, specifically, inflammatory gastrointestinal disease, for example, inflammatory bowel disease, or predicting or evaluating responsiveness of prevention, improvement or treatment to gastrointestinal disease, for example, inflammatory bowel disease, comprising detecting a *Faecalibacterium cancerinhibens* in a biological sample of a test subject.

The present invention provides a method for prevention, improvement or treatment of disease or disorder associated with the reduced level of *Faecalibacterium cancerinhibens* compared to the level of *Faecalibacterium cancerinhibens* in a healthy control group, wherein the method includes determining a subject as having a reduced level of *Faecalibacterium cancerinhibens,* and administering a composition including *Faecalibacterium cancerinhibens* when the subject is determined to have a reduced level of *Faecalibacterium cancerinhibens.*

An embodiment of the present invention relates to an anti-inflammatory agent, comprising at least one selected from the group consisting of; a microbial cell of a *Faecalibacterium* spp.; a culture of the microorganism; a lysate of the microorganism; and at least an extract selected from the group consisting of the microbial cell, culture and lysate. The *Faecalibacterium* spp. has an anti-inflammatory activity.

The composition may be a pharmaceutical composition or food composition, and may include probiotics alone, or in combination with prebiotics.

Herein, the inflammatory disease may include various inflammations, and for example, it may be inflammatory gastrointestinal disease, preferably, inflammatory gastrointestinal disease selected from the group consisting of inflammatory bowel disease (Crohn's disease, ulcerative colitis), irritable bowel syndrome, chronic digestive disorder (coeliac disease), infectious colitis (for example, caused by C. difficile), Behcet's disease, other gastrointestinal-related diseases, and any combination thereof. Otherwise, the inflammatory disease is inflammatory autoimmune disease, and herein, dysbiosis-balanced microbiota and low-grade mucosal inflammation may be related to an etiologic cause of disease, such as diabetes (types 1 and 2), asthma and atopic disorders.

In the present invention, the effect of improvement, treatment or prevention of immunomodulatory or inflammatory disease exhibited by the strain of present invention may be characterized by being induced by increase or decrease of cytokines involved in inflammation inhibition and immunomodulation, specifically, any one or more actions of decrease in secretion of IFN-$\gamma$, IL-8, IL-6 and TNF-$\alpha$ and the like, and increase in secretion of IL-10.

The role of IFN-$\gamma$ is representatively NK cell activation, macrophage activation and increased expression of MHC and the like, and chronic exposure to IFN-$\gamma$ is known to be involved in several non-infectious pathologies such as autoimmune disease and inflammatory disease.

When inflammation occurs, Neutrophil can migrate to intestinal lumen through tight junction between intestinal epithelial cells and can play a role in additionally exacerbating inflammation. In this stage, IL-8 as an inflammatory cytokine is an important factor playing a role in attracting such Neutrophil to the inflammatory site, and causing tissue damage by infiltrating Neutrophil into the lamina propria musoca and secreting inflammatory mediators.

IL-6 is secreted from various cells such as T lymphocytes, macrophages and the like to stimulate an immune response, and in this case, it may cause tissue damage leading to infection, trauma, particularly, burns or inflammation. In addition, it is known that IL-6 can play a role in accelerating inflammation by inducing Th17 cell differentiation and promoting secretion of ROR$\gamma$t or IL-17.

TNF-$\alpha$, one of major factors causing inflammation, is mainly secreted from activated macrophages, and is secreted from various cells such as helper T cells, natural killer cells, and damaged neurons and the like. In addition, its most important role is regulation of immunocytes, which can induce production of IL-1 and IL-6, and is known to play a role in promoting an inflammatory response in various inflammatory diseases such as rheumatoid arthritis, Crohn's disease and ulcerative colitis, and the like.

In addition, IL-10 is an important immunomodulatory cytokine produced in various cells such as helper T cells, B cells, monocytes and the like, and its most important function is inhibition of an inflammatory response, and it has immunosuppressive and anti-inflammatory activities such as inhibiting production of pro-inflammatory cytokines including IL-6 and TNF-$\alpha$, and the like.

Inflammatory bowel disease (IBD) is chronic inflammatory disease of the gastrointestinal tract characterized by diarrhea, bloody stool, abdominal pain and weight loss, and is chronic, recurrent inflammation of unknown etiology. IBD includes ulcerative colitis (UC) and Crohn's disease (CD), which are two major disease conditions, and more specifically, includes chronic or acute ulcerative colitis and Crohn's disease. UC mainly affects the mucosal layer of large intestine or colon. In contrast, CD is defined as transmural granulomatous inflammation that may include segments of small and large intestine.

The present invention provides an anti-inflammatory (probiotic) composition, for example, a functional food, neutraceuticals or a pharmaceutical composition containing probiotic bacteria, and a method for promoting survival of probiotics in the gastrointestinal tract.

In the present invention, the *Faecalibacterium* spp. may be for example, a bacterium comprising a 16S rDNA sequence having sequence identity of 98% or more, 98.5% or more, 99% or more, 99.5% or more, 99.8% or more, or 99.9% or more with the nucleotide sequence of SEQ ID NO: 1, and may be a bacterium comprising a 16S rDNA gene comprising the nucleotide sequence of SEQ ID NO: 1, preferably, the *Faecalibacterium cancerinhibens* strain CLCC1 deposited with Accession number KCTC 13783BP. The microorganism or bacterium may have at least one characteristic selected from the group consisting of the following characteristics:

(1) 16S rRNA comprising a nucleotide sequence of SEQ ID NO: 1 or 16S rRNA comprising a nucleotide sequence having a nucleotide sequence identity of 98% or more, 98.5% or more, 99% or more, 99.5% or more, 99.8% or more or 99.9% or more;

(2) a lipid production pattern, specifically, not including 17:0 iso 3OH fatty acid, more specifically, including at least one fatty acid selected from the group consisting of 15:1 $\omega$8c fatty acid, 19:0 cyclo $\omega$10c/19$\omega$6 fatty acid, 20:1 $\omega$9c fatty acid, 14:0 3OH/16:1 iso I fatty acid, 15:0 3OH fatty acid, 16:0 iso fatty acid and 16:0 iso 3OH fatty acid, and comprising PL3 which is not present in *Faecalibacterium prausnitzii*;

(3) an average nucleotide identity (ANI) value of 95% or less, compared to the genome sequence of the *Faecalibacterium cancerinhibens* strain CLCC1.

(4) an immunomodulating activity and/or an anti-inflammatory activity, for example, reducing the level of at least one inflammatory cytokine selected from the group consisting of IFN-γ, IL-6, IL-8 and TNF-α, and/or increasing the level of at least one anti-inflammatory cytokine selected from the group consisting of IL-10 and TNF-β, so as to have an anti-inflammatory activity and an immunomodulatory activity, for example, an immunosuppressive activity;

(5) an anti-cancer activity, specifically, an activity of preventing cancer occurrence, delaying or inhibiting the tumor growth, reducing the tumor size or volume, or inhibiting or delaying cancer metastasis, for example, an activity of reducing the tumor volume by 10 to 90 % compared to the control group not administered with the microorganism, when *Faecalibacterium cancerinhibens* is administered into mice subcutaneously transplanted with a cancer cell line;

more specifically, an inhibitory activity of tumor growth or an anti-cancer activity of causing apoptosis of cancer cells;

a relative reduction rate of tumor volume of 5% or more compared to a control group not administered with the microorganism, when being administered into an animal model transplanted with a cancer cell line; or

a tumor growth inhibition rate of the tumor weight of 5% or more compared to a control group not administered with a microorganism, when being administered into an animal model transplanted with a cancer cell line;

(6) being cultured at a culture temperature of 20 to 40 °C, or under an anaerobic condition, specifically, being cultured at a temperature of 20 to 40 °C, 25 to 40 °C, 30 to 40 °C, 35 to 40 °C or 37°C; and

(7) a high content of butyric acid, specifically, 2 to 50 times higher than that of *Faecalibacterium prausnitzii* ATCC 27768 strain.

*Faecalibacterium cancerinhibens* of the present invention may have at least one characteristic selected from the above characteristics. For example, the strain having all the characteristics of (1) to (7) above, and *Faecalibacterium cancerinhibens* CLCC1 is a new species that has not been previously isolated and reported. The *Faecalibacterium* spp. strain was identified by the above method, named as *Faecalibacterium cancerinhibens* CLCC1, and deposited to Korea Research Institute of Bioscience and Biotechnology, Korean Collection for Type Cultures as Accession number of KCTC 13783BP.

More specifically, as a result of genome analysis of *Faecalibacterium cancerinhibens* strain CLCC1 and comparison with allied species, *Faecalibacterium cancerinhibens* strain CLCC1 shows an average nucleotide identity (ANI) value of 85.99% at a genome level with the closest allied species, *Faecalibacterium prausnitzii,* so it is a novel species that has not been previously isolated and reported.

More specifically, the anti-cancer activity according to the present invention may be an anti-cancer activity against colorectal cancer and/or liver cancer, and for example, it may be an activity to inhibit occurrence or progress of liver cancer and/or colorectal cancer. Specifically, it may delay tumor occurrence or inhibit the growth rate of tumor, and it may further have an activity to prevent cancer metastasis due to inhibition of the growth rate of tumor.

*Faecalibacterium* spp. according to the present invention is isolated from feces of healthy adult male human, the effect of microorganism to inhibit cancer occurrence and tumor growth in a human cancer cell line, a mouse model subcutaneously transplanted with the cancer cell line, or a mouse model having the corresponding tissue transplanted with the cancer cell line. Specifically, in an example of the present invention, *Faecalibacterium cancerinhibins* strain CLCC1 was isolated from feces of healthy adult males and was confirmed to have an effect of inhibiting tumor growth in mice which was induced for colorectal or liver cancer or transplanted with colorectal or liver cancer. The activity to inhibit the tumor growth may be measured by using for example, the tumor volume/size or tumor weight.

When the *Faecalibacterium cancerinhibens* provided by the present invention is administered to the animal model subcutaneously transplanted with the cancer cell line, compared to the control group not administered by the microorganism, the tumor growth inhibition rate according to the tumor weight may be 5% or more, 10% or more, 15% or more, 20% or more, 25% or more, or 30% or more, and the tumor growth inhibition rate according to the tumor weight may be calculated by following Equation 1.

[Equation 1]

$$IR(\%) = (1-(T1/C1)) \times 100$$

In the Equation 1, IR is the tumor growth inhibition rate according to the tumor weight (IR), and T1 is the average tumor weight in each experimental group, and C1 is the average tumor weight in the negative control group.

When *Faecalibacterium cancerinhibens* provided by the present invention is administered to the animal model subcutaneously transplanted with a cancer cell line, compared to the control group not administered by the microorganism, the relative reduction rate of tumor volume may be 5% or more, 10% or more, 15% or more, 20% or more, 25% or more, or 30% or more, and the tumor growth inhibition rate according to the tumor volume may be calculated by Equation 2 below.

[Equation 2]

$$\text{Relative reduction rate of tumor volume (\%)} = (1-(T2/C2)) \times 100$$

In the Equation 2, T2 is the average tumor volume in each experimental group and C2 is the average tumor volume in the negative control group.

When *Faecalibacterium cancerinhibens* strain CLCC1 is administered to a tumor animal model transplanted with cancer cell lines according to an example of the present invention, based on 100% of the tumor volume of the negative control group not administered with the microorganism, the tumor volume may be 90% or less, 85% or less, 80% or less, 77% or less, 70% or less, 67% or less, and the tumor volume may have a numerical range combining a value selected from the lower limit of 10% or more, 15% or more, 18% or more, and 20% or more, and a value selected from the upper limit of 90% or less, 85% or less, 80% or less, 77% or less, 70% or less, and 67% or less.

In case that the cancer is colorectal cancer, *Faecalibacterium cancerinhibens* strain CLCC1 may have an activity to reduce the tumor volume by a level of 10 to 90%, 10 to 85%, 10 to 80%, 10 to 77%, 10 to 70%, 10 to 67%, 20 to 90%, 20 to 80%, 20 to 70%, 20 to 67%, 30 to 90%, 30 to 80%, 30 to 70%, 30 to 67%, 40 to 90%, 40 to 80%, 40 to 70%, 40 to 67%, 50 to 90%, 50 to 80%, 50 to 70%, 50 to 67% or 60 to 67%, based on 100% of the tumor volume of the negative control group not administered with the microorganism, when the microorganism is administered.

Specifically, it was confirmed that the average tumor size at the day (Day 1) when the administration of the microorganism was started in a mouse subcutaneous tumor model using a human colorectal cancer cell line had a tumor volume of about 60 to 67% compared to the negative control group not administered with the microorganism, thereby confirmed that the effect of *Faecalibacterium cancerinhibens* on inhibiting the growth of colorectal cancer.

In case that the cancer is liver cancer, *Faecalibacterium cancerinhibens* CLCC1 provided by the present invention may have an activity to reduce the tumor volume by a level of 90% or less, 85% or less, 80% or less, 77% or less, 10 to 90%, 10 to 85%, 10 to 80%. 10 to 77%, 15 to 90%, 15 to 85%, 15 to 80%, 15 to 77%, 18 to 90%, 18 to 85%, 18 to 80%, or 18 to 77%, based on 100% of the liver tumor volume of the control group not administered with the microorganism, when the microorganism was administered.

In the tumor volume reduction of liver cancer according to the present invention, the volume of the liver cancer was 19.5% to 75.3%, and the average volume of the liver cancer was about 32.9% lower in the experimental groups orally administered with CLCC1, compared to the control group. Thus, it can be found that the orally administered CLCC1 microorganism has an activity to inhibit the occurrence and progression of the tumor in the liver cancer.

The *Faecalibacterium cancerinhibens* according to the present invention may have a characteristic showing a lipid production pattern different from *Faecalibacterium prausnitzii* which is a *Faecalibacterium* spp.

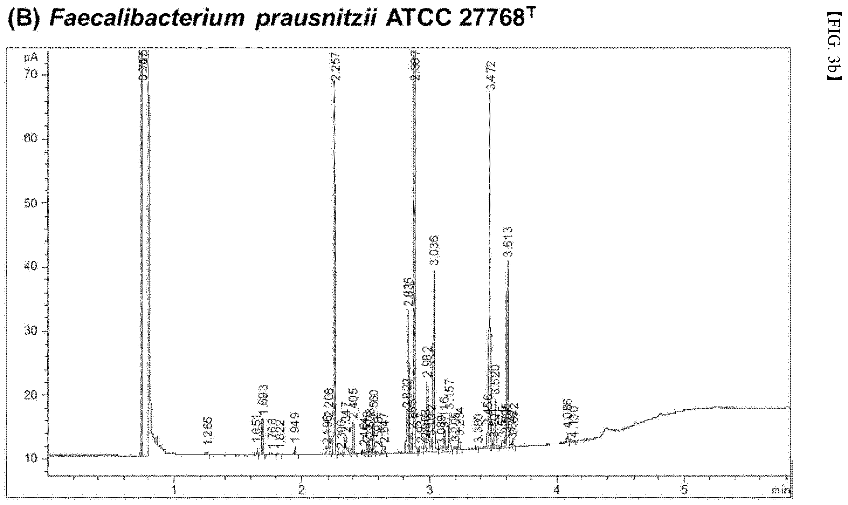

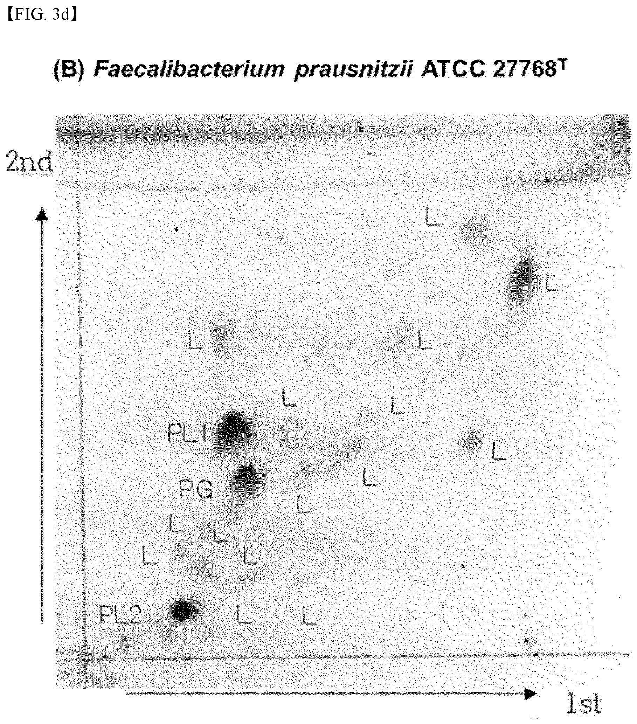

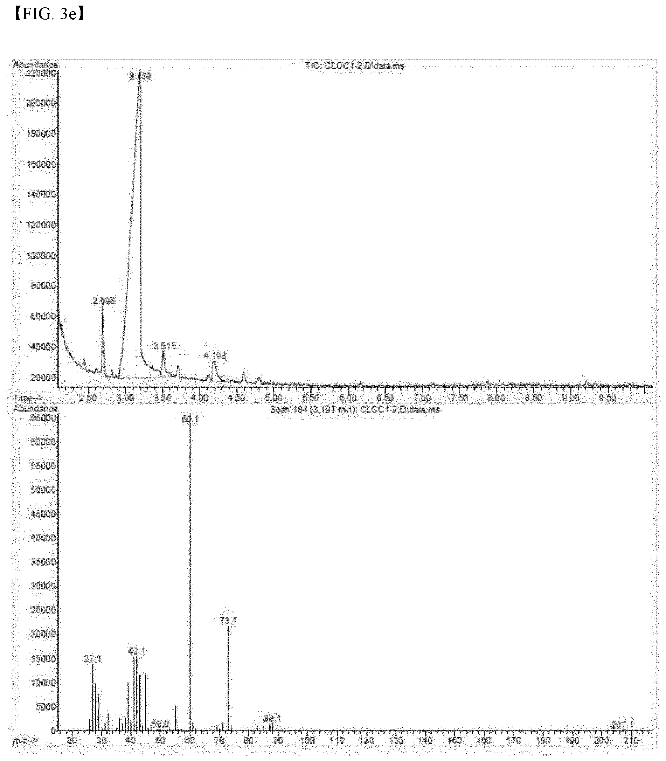

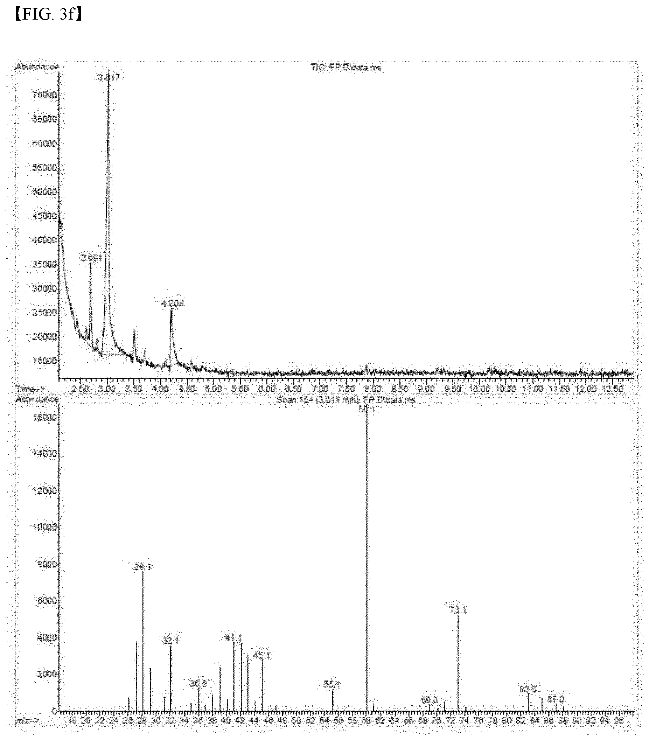

The *Faecalibacterium cancerinhibens* according to the present invention does not comprise 17:0 iso 3OH fatty acid. In an example of the present invention, as a result of analyzing the polar lipid content of *Faecalibacterium cancerinhibens* CLCC1 and *Faecalibacterium prausnitzii,* it was confirmed that CLCC1 additionally had PL3 which is absent in *Faecalibacterium prausnitzii* (FIG. 3b). The *Faecalibacterium cancerinhibens* according to the present invention may comprise one or more fatty acids selected from the group consisting of 15:1 ω8c fatty acid, 19:0 cyclo ω10c/19ω6 fatty acid, 20:1 ω9c fatty acid, 14:0 3OH/16:1 iso I fatty acid, 15:0 3OH fatty acid, 16:0 iso fatty acid and 16:0 iso 3OH fatty acid.

In an example of the present invention, as a result of comparing the fatty acid composition of *Faecalibacterium cancerinhibens* CLCC1 and *Faecalibacterium prausnitzii* by performing gas chromatography analysis, it could be seen that there was a difference in the composition of major fatty acids between two microorganisms. Specifically, 15:1 ω8c fatty acid, 19:0 cyclo ω10c/19ω6 fatty acid, 20:1 ω9c fatty acid, 14:0 3OH/16:1 iso I fatty acid, 15:0 3OH fatty acid, 16:0 iso fatty acid and 16:0 iso 3OH fatty acid were detected only in the CLCC1 microorganism, but 17:0 iso 3OH fatty acid was detected only in *Faecalibacterium prausnitzii* ATCC 27768.

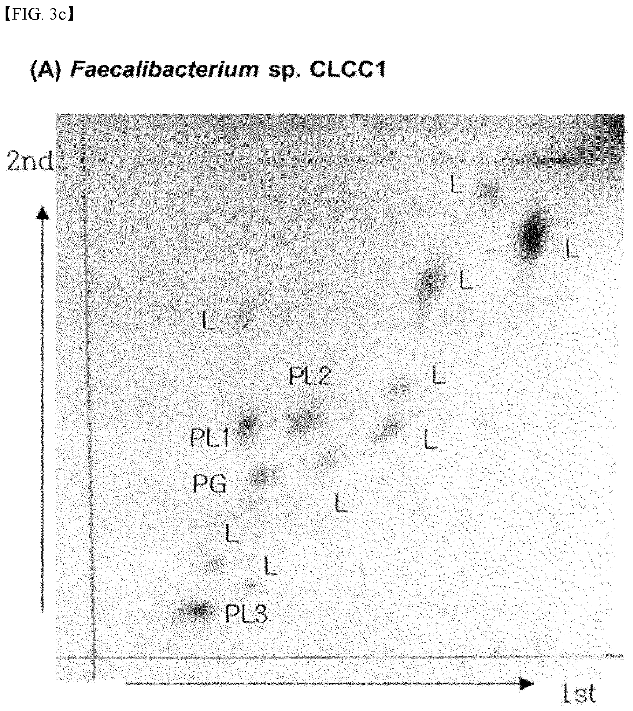

The *Faecalibacterium cancerinhibens* provided by the present invention has the high production of butyric acid among short chain fatty acids (SCFA). Specifically, the butyric acid production of *Faecalibacterium cancerinhibens* strain CLCC1 of the present invention may be 2 times or more, 3 times or more, 4 times or more, 5 times or more, 6 times or more, 6.5 times or more, or 7 times or more, compared to the *Faecalibacterium prausnitzii* species, and specifically, it may be 2 to 50 times, 2 to 40 times, 2 to 30 times, 2 to 20 times, 2 to 15 times, 2 to 10 times, 5 to 50 times, 5 to 40 times, 5 to 30 times, 5 to 20 times, 5 to 15 times, or 5 to 10 times. In one specific embodiment, as a result of SCFA analysis of the culture supernatant of *Faecalibacterium cancerinhibens* strain CLCC1 and *Faecalibacterium prausnitzii,* the butyric acid content of *Faecalibacterium cancerinhibens* strain CLCC1 showed 7.8 times higher than that of *Faecalibacterium prausnitzii* (FIGs. 3c to 3d).

The *Faecalibacterium cancerinhibens* strain according to the present invention forms a colony within 3mm or within 2mm and has a long rod-shaped cell shape when observed by optical microscope, when smeared and cultured on an agar medium. In an example of the present invention, *Faecalibacterium cancerinhibens* had a genome size of 2.9Mbp, and the number of protein coding regions (CDS) was 2695, and the GC ratio was 56.1% and the number of rRNA genes was 21, and the total number of tRNA genes was 69. In *Faecalibacterium cancerinhibens* CLCC1 according to the present invention, the butyric acid (butanoic acid) was detected at a content 7.8 times higher than that of *Faecalibacterium prausnitzii* was detected (FIGs. 3c to 3d).

In a specific embodiment of the present invention, the colitis model induced by DSS is historically similar to human ulcerative colitis (UC) in terms of changes and loss of crypt morphology, ulceration, inflammatory cell infiltration and the like, and is widely used in colitis models. The difference according to the dose by administering the *Faecalibacterium cancerinhibens* CLCC1 according to the present invention at a dose of $2 \times 10^7$ cells/head and $2 \times 10^8$ cells/head at a frequency of once/day for 24 days, the difference according to the administration period by administering it at a dose of $2 \times 10^8$ cells/head at a frequency of once/day for 10 days, and the effect on colitis improvement according to live and dead cells by administering it at a dose of dead cell $2 \times 10^8$ cells/head at a frequency of once/day for 24 hours were confirmed.

As a result, the body weight of the negative control group, in which colitis was induced with 3% DSS, decreased rapidly, and soft stools and diarrhea including bloody stools were observed, and the length of the large intestine was reduced, and histopathologically, the crypt structure was disrupted and Goblet cells involved in mucosal formation disappeared, and full-thickness inflammation occurred, and inflammatory cells were observed. In addition, in the negative control group, the expression of inflammatory cytokines, IFN-$\gamma$, TNF-$\alpha$ and IL-6 was increased.

Repeated administration of *Faecalibacterium cancerinhibens* CLCC1 living cells to an animal model of DSS-induced colitis in BALB/c mice reduced colitis-induced weight loss, improved the decrease in the length of the large intestine, and reduced tissue damage to the colorectal mucosa, and inhibited the expression of the inflammatory cytokines, IFN-$\gamma$, TNF-$\alpha$, and IL-6, thereby having an effect on improvement of colitis. Specifically, the expression of the inflammatory cytokines, IFN-$\gamma$ and IL-6 was statistically significantly reduced compared to the negative control group, and TNF-$\alpha$ also showed a tendency to decrease. A similar result was obtained for all measurements, without significant change according to the number of bacteria and duration of administration. The weight loss and length of the large intestine of the group administered with dead cell $2 \times 10^8$ cells/head 24 times was similar to that of the negative control group. In addition, the inflammatory cytokines, IFN-$\gamma$ and IL-6 were reduced compared to the negative control group.

The composition for preventing, improving or treating an inflammatory gastrointestinal tract, for example, IBD according to the present invention may comprise a microbial cell itself, or be a cell-free form not comprising a microbial cell. The lysate means a lysate obtained by crushing the *Faecalibacterium* spp. microbial cells or a supernatant obtained by centrifugation of the lysate. Herein, unless otherwise stated, the *Faecalibacterium* spp. having an anti-cancer activity is used to mean one or more kinds selected from the group consisting of a microbial cell of the microorganism; a culture of the microorganism; a lysate of the microorganism; and an extract of one or more kinds selected from the group consisting of the microbial cell, culture and lysate.

The composition according to the present invention may comprise a lyophilized microbial cell. The lyophilization of the microbial cell of the microorganism may be carried out by a method known to those skilled in the art. Alternatively, the composition of the present invention may comprise a culture of a live microorganism. In an embodiment, the microorganism of the present invention may be a living cell, a dead cell, or a mixture thereof when the *Faecalibacterium* spp. itself comprises a microbial cell.

The composition for preventing, improving or treating IBD according to the present invention comprises the microorganism of the present invention in a therapeutically effective amount. The microorganism in a therapeutically effective amount is sufficient for exhibiting a beneficial effect to a patient. The bacterium or extract is formulated to be administered to a subject in a therapeutically effective amount, depending on for example, the type of subject, severity of disease and route of administration. For example, an appropriate daily dose of the bacterium for an adult human may be about $1 \times 10^3$ to about $1 \times 10^{11}$ colony forming unit (CFU), for example, about $1 \times 10^3$ to about $1 \times 10^{15}$ CFU, about $1 \times 10^3$ to about $1 \times 10^{14}$ CFU, about $1 \times 10^3$ to about $1 \times 10^{13}$ CFU, about $1 \times 10^3$ to about $1 \times 10^{12}$ CFU, about $1 \times 10^3$ to about $1 \times 10^{11}$ CFU, about $1 \times 10^5$ to about $1 \times 10^{15}$ CFU, about $1 \times 10^5$ to about $1 \times 10^{14}$ CFU, about $1 \times 10^5$ to about $1 \times 10^{13}$ CFU, about $1 \times 10^5$ to about $1 \times 10^{12}$ CFU, about $1 \times 10^5$ to about $1 \times 10^{11}$ CFU, about $1 \times 10^7$ to about $1 \times 10^{15}$ CFU, about $1 \times 10^7$ to about $1 \times 10^{14}$ CFU, about $1 \times 10^7$ to about $1 \times 10^{13}$ CFU, about $1 \times 10^7$ to about $1 \times 10^{12}$ CFU, about $1 \times 10^7$ to about $1 \times 10^{11}$ CFU, and the unit of the bacterium dose may be /g colony forming unit (CFU), that is, CFU/g, based on the total weight of the composition for preventing, improving or treating an inflammatory gastrointestinal tract, for example, IBD.

Administration of the bacterium may be by any appropriate route. For example, the specific *Faecalibacterium* spp. of the present invention may be administered to an animal (including humans) in an orally digestible form. In the case of the food composition or neutraceuticals, the bacterium may be simply comprised in a conventional food product or food supplement. A representative pharmaceutical formation includes capsules, microcapsules, tablets, granules, powders, troches, pills, suppositories, suspensions and syrups. In another embodiment, the composition is in a form for rectal administration to an animal (including humans) as rectal suppository or enema. An appropriate formulation may be prepared by a conventional method using conventional organic and inorganic additives. The amount of the active ingredient in the medical composition may be at a level that achieves the desired therapeutic effect.

The composition of the present invention may be encapsulated for transport of the *Faecalibacterium* spp. in the intestine. The encapsulation protects the composition until it is transported to the target site, for example, by rupture by chemical or physical stimuli, such as physical disruption that may be caused by changes in pressure, enzymatic activity or pH. Any appropriate method for encapsulation may be used. An illustrative encapsulation technology includes capture in porous matrix, attachment or adhesion to the surface of a solid carrier, aggregation or autoagglutination by a crosslinking agent, and mechanical blocking after microporous membranes or microcapsules.

The pharmaceutical composition for prevention, improvement or treatment of inflammatory disease, for example, inflammatory gastrointestinal disease according to the present invention comprises at least one selected from the group consisting of; a microbial cell of a *Faecalibacterium* spp.; a culture of the microorganism; a lysate of the microorganism; and at least an extract selected from the group consisting of the microbial cell, culture and lysate as an active ingredient, and may comprise an acceptable carrier, diluent or excipient. The acceptable carrier or diluent for therapeutic use is well known in the pharmaceutical field.

The dose of the pharmaceutical composition of the present invention may vary depending on the patient's age, body weight, gender, administration type, health condition and disease degree, and it may be administered in divided doses from once to several times a day at a certain time interval according to the judgement of the doctor or pharmacist. For example, based on the active ingredient content, the daily dose may be 0.1 to 500 mg/kg, preferably, 0.5 to 300 mg/kg. The above dose is an example of an average case, and the dose may be higher or lower depending on individual differences.

The composition of the present invention may be probiotics, and the probiotics may be mixed with at least one appropriate prebiotic compound. The prebiotic compound is generally a non-digestible carbohydrate such as an oligo- or polysaccharide or sugar alcohol, which is not degraded or absorbed in the upper digestive tract. Known prebiotics include commercially available products such as inulin and transgalacto-oligosaccharides. Synbiotics represent nutritional supplements that combine probiotics and prebiotics in a synergistic form.

An embodiment of the present invention relates to a food composition for prevention or improvement of inflammatory bowel disease, for example, a food composition or anti-cancer functional food composition for prevention or improvement of inflammatory bowel disease, comprising at least one selected from the group consisting of; a microbial cell of a *Faecalibacterium* spp.; a culture of the microorganism; a lysate of the microorganism; and at least an extract selected from the group consisting of the microbial cell, culture and lysate.

It is intended to include all foods, food additives, health functional foods, beverages and beverage additives, and the like in a conventional sense. Herein, the beverage means a generic term for a drink for thirst quenching or taste enjoyment and is intended to include functional beverages. The beverage may be in liquid, syrup and/or gel form.

As an active ingredient contained in the food composition, the content of the *Faecalibacterium* spp. is not particularly limited, suitably depending on the type of food, desired use, and the like, and for example, at least one selected from the group consisting of a microbial cell of the *Faecalibacterium* spp., a culture of the microorganism, a lysate of the microorganism and an extract of the microorganism, which are active ingredients of the total of the food, may be added in an amount of 0.00001 % by weight to 100 % by weight, 0.001 % by weight to 99.9 % by weight, 0.1 % by weight to 99 % by weight, more preferably, 1 % by weight to 50 % by weight, 0.01 to 15 % by weight. For example, the food composition may be added in a ratio of 0.02 to 10 g, preferably, 0.3 to 1 g, based on 100 ml.

The composition of the present invention may be formulated. For example, food may provide nutritional benefits in addition to the therapeutic effect of the present invention as a nutritional supplement.

Herein, the term "prediction of risk" or "prediction of likelihood of occurrence" refers to determining whether a subject has a possibility of developing a specific disease, and it may be used clinically to delay or prevent the occurrence of a patient with a high risk of occurrence of a specific disease by special and appropriate management, or to decide on treatment by selecting the most appropriate treatment method. In addition, "diagnosis" means confirming the presence or characteristics of a pathological state, and for the purpose of the present invention, diagnosis may mean confirming the occurrence of a disease.

The "subject" refers to a subject for predicting a risk of occurrence of inflammatory bowel disease or diagnosis of cancer, predicting or evaluating treatment response to a *Faecalibacterium* spp., or with the possibility of administering the microorganism, or a subject in need of receiving an administration of the microorganism and monitoring the efficacy of the microorganism, and for example, it may be a subject suspected of being at risk of occurrence of inflammatory bowel disease, a subject receiving a diagnosis of inflammatory bowel disease, or a subject receiving treatment for anti-inflammatory bowel disease. The subject is preferably a human.

In the present description, a biological sample of a subject may include a cell extract, any organ, tissue, cell, saliva or feces, or the like isolated from a subject, such as a sample isolated from a mammal having inflammatory bowel disease or suspected of having inflammatory bowel disease. For example, the sample may include, without limitation, a cell or tissue from any part (including without limitation colon, stomach, feces, anus, rectum, and duodenum) of the gastrointestinal tract (for example, from biopsy or autopsy), a cell lysate of stomach obtained from a patient (human or animal), an experimental subject or experimental animal, or an excretion from a subject. Preferably, the sample may be feces.

In an embodiment of the present invention, detecting the *Faecalibacterium cancerinhibens* includes confirming the presence or absence of the microorganism in a sample, identifying the microorganism, measuring the level of the microorganism, and analyzing relative occupancy. Specifically, detection of *Faecalibacterium cancerinhibens* or measurement of the level of the microorganism in a sample from a test subject, may include performing using a nucleic acid sequence, amino acid sequence, metabolite, or the microorganism itself as a biomarker specific for the *Faecalibacterium cancerinhibens,* or evaluating the level of the microorganism by obtaining sequence information of the whole genome, or at least one specific gene in the microbial community present in the sample.

Specifically, after constructing a sequencing library using the PacBio sequencing library kit, whole genome sequencing may be performed using the PacBio RS II platform, and genome sequencing information may be analyzed using Whole Genome analysis pipeline of BIOiPLUG, our genome data analysis platform, and as a result, the whole genome size of the *Faecalibacterium cancerinhibens* strain CLCC1 is about 2.9Mbp, and the number of protein coding regions (CDS) is 2695, and a region specific to *Faecalibacterium cancerinhibens* such as CDS region or Intron region or the like is selected and used as a biomarker.

For example, the detection of the microorganism may be performed by calculating relative abundance of microorganisms distinguished at the genus or species level with respect to the intestinal microbial community of the test subject and *Faecalibacterium cancerinhibens* in these microorganisms, and measuring the level of *Faecalibacterium cancerinhibens.*

The measurement of the level of the microorganism, may be the measurement of the ratio of the *Faecalibacterium cancerinhibens* strain from a sample of a subject, for example, intestine biopsis or a feces sample. The method for measuring the ratio of the strain may be appropriately selected and used by those skilled in the art according to the technical common sense of the art, and for example, it may be performed using a molecular method selected from the group consisting of quantitative polymerase chain reaction (qPCR), massively parallel sequencing, fluorescence in-situ hybridization (FISH), microarray and PCR-ELISA, but not limited thereto.

For example, it may determine that the test subject is at risk of developing bowel disease, when the test subject's level of the microorganism is lower than reference subject's level of the microorganism, by measuring the level of the *Faecalibacterium cancerinhibens* microorganism in the subject's sample and comparing it to the level of the microorganism in a reference subject.

The method may further comprise determining that the test subject is at risk of developing bowel disease, when the test subject's level of the microorganism is lower than the reference subject's level of the microorganism by measuring the *Faecalibacterium cancerinhibens* microorganism level in the test subject's sample and comparing the microorganism level with that of a reference subject.

By predicting a risk of developing cancer in advance by metagenome analysis using a human-derived sample according to the present invention, the risk group of cancer can be diagnosed and predicted early, and the onset time can be delayed or the onset can be prevented by appropriate management, and even after the onset, the incidence rate can be lowered and the therapeutic effect can be increased. In addition, by avoiding exposure to causative factors through metagenome analysis in patients diagnosed with cancer, the course of the cancer can be improved, or recurrence can be prevented.

The method for detecting *Faecalibacterium cancerinhibens* in a sample from a test subj ect or measuring the level of the microorganism will be described in more detail below.

As an embodiment of the present invention, a method for detecting the microorganism or measuring the level using g a specific gene, for example, a 16S rRNA gene sequence may be provided.

Specifically, the level of the microorganism may be preferably measured by determining the level of a specific nucleic acid sequence in the sample, and the nucleic acid sequence may be preferably a 16S rRNA gene sequence of one or more bacterium above, more preferably, a region of the 16S rRNA gene sequence, for example, at least one of variable regions V1 and/or V6 of the 16S rRNA gene sequence.

Accordingly, the detection of the microorganism may be carried out by obtaining the microbial species distinguished at the species level with respect to the intestinal microbial community of the test subject and the relative abundance of *Faecalibacterium cancerinhibens* in these microbial species, and measuring the relative level of *Faecalibacterium cancerinhibens.* It may comprise obtaining 16S rRNA genetic information from the genome information of the intestinal microorganism; and analyzing the 16S rRNA information of the intestinal microorganism and obtaining the abundance of the microbial species comprised in the intestinal microbial community of the test subject.

The genome information of the test subject may be genetic information stored in a storage medium, or genome information of a microorganism isolated from a sample of the test subject. The obtaining 16S rRNA genetic information, may be analyzing the 16S rRNA gene sequence of the extracted DNA, using a next-generation genome sequencing (NGS) platform.

The analyzing the 16S rRNA gene sequence of the extracted DNA may comprise performing PCR using a primer set capable of specifically amplifying a variable region of 16S rRNA, preferably, performing PCR using a primer set capable of specifically amplifying V3 to V4 regions of 16S rRNA, more specifically, performing PCR using a universal primer having the following sequence to produce an amplicon.

By analyzing the 16S rRNA information of the intestinal microorganism, the intestinal microbial community information which obtains the microbial species distinguished at the species level with respect to the intestinal microbial community of the test subject and the abundance of these microbial species may be analyzed.

The analyzing the microbial community, may comprise identification and classification of microorganisms at the genus or species level using 16S rRNA database and/or analysis of each microbial community population. The database used for identification and classification of the microorganisms may be appropriately selected and used by those skilled in the art if necessary, and for example, it may be at least one database selected from the group consisting of EzBioCloud, SILVA, RDP and Greengene, but not limited thereto.

The microbial community population may be expressed as a ratio (%) occupied by a specific microbial community in the total intestinal microbial flora. The ratio (%) occupied by the microbial community may be expressed as a percentage of the frequency of the number of 16S rRNA reads of a specific microorganism among the total number of sequencing reads. The specific microorganism is *Faecalibacterium cancerinhibens* provided by the present invention.

When which microorganism exists is to be known by performing microbial community analysis, the cost and time of analysis may be reduced by using an amplicon sequencing method that selectively amplifies only a marker gene for species identification and then analyzes the nucleotide sequence of the amplification product, the cost and time required for analysis may be reduced. For bacterial community analysis, the 16S rRNA gene is used as a marker gene, and this gene is present in all bacteria and appropriately comprises conserved regions and mutated regions (v1-v9), so it is suitable for phylogenetic analysis and ecological research.

As an additional embodiment of the present invention, a method is provided for detecting the microorganism or measuring the level using a biomarker specific for *Faecalibacterium cancerinhibens.*

In the present invention, as a biomarker specific for the *Faecalibacterium cancerinhibens,* it may comprise a nucleic acid sequence, an amino acid sequence and a metabolite. Accordingly, as an agent capable of detecting the biomarker of the present invention, a primer, a probe, an antisense oligonucleotide, an aptamer and an antibody and the like, which can specifically detect an organic biomolecule such as a protein, nucleic acid, lipid, glycolipid, glycoprotein or sugar (monosaccharide, disaccharide, oligosaccharide, etc.) which is specifically present in the corresponding microorganisms in a sample may be used.

As an embodiment, in the present invention, the agent capable of detecting the microorganisms may be a probe capable of detecting the corresponding microorganism, or a primer for amplifying the probe. It is preferred that the primer to specifically detects a specific probe gene of the corresponding microorganisms and does not specifically bind to a genome sequence of another microorganism.

One specific embodiment of the present invention relates to a probe, a primer set, a kit, a composition and a method for detecting *Faecalibacterium cancerinhibens* capable of specifically detecting *Faecalibacterium cancerinhibens* by distinguishing it from other microbial species or subspecies, and sensitively detecting *Faecalibacterium cancerinhibens* present in a small amount in a sample. An example of the probe for detecting *Faecalibacterium cancerinhibens* may be Site-specific DNA-methyltransferase (dam), but not limited thereto. In addition, the probe may be linked to a detection label. The detection label may be any chemical moiety that may be detected by any method known in the art. The primer of the present invention may also be modified using a label capable of directly or indirectly providing a detectable signal. The example of the label includes radioactive isotopes, fluorescent molecules, biotin, and the like. As a method for amplifying a sequence using a primer, various methods known in the art may be used.

In an embodiment of the present invention, the analyzing may perform at least one method selected from the group consisting of gel electrophoresis, capillary electrophoresis, sequencing, DNA chip, radioactivity measurement, fluorescence measurement and phosphorescence measurement.

[ADVANTAGEOUS EFFECTS]

The present invention relates to a composition for diagnosis or treatment of inflammatory disease, for example, inflammatory gastrointestinal disease, comprising a *Faecalibacterium* spp..

[BRIEF DESCRIPTION OF THE DRAWINGS]

FIG. 1a is a photograph showing the colon form formed after culturing *Faecalibacterium cancerinhibens* CLCC1 in Reinforced clostridial media (RCM) medium for 3 days, and a photograph of the cell appearance observed with an optical microscope in an exponential growth phase during liquid culture.

FIG. 1b is a photograph taken with a scanning electron microscope of cells in an exponential growth phase during liquid culture of *Faecalibacterium cancerinhibens* CLCC1.

FIG. 2a is a diagram showing the phylogenetic position obtained through 16S rRNA analysis of *Faecalibacterium cancerinhibens* CLCC1.

FIG. 2b is a diagram showing the phylogenetic position using 92 core genes present in the genome of *Faecalibacterium cancerinhibens* CLCC1.

FIG. 2c is a relationship diagram of *Faecalibacterium cancerinhibens* strain CLCC1 and allied species based on average nucleotide identity (ANI) values.

FIG. 2d is a table comparing ANI values of *Faecalibacterium cancerinhibens* CLCC1 and allied species.

FIG. 3a is a graph of the result of gas chromatography analysis showing the fatty acid composition of the *Faecalibacterium cancerinhibens* strain CLCC1.

FIG. 3b is a graph of the result of gas chromatography analysis showing the fatty acid composition of the *Faecalibacterium prausnitzii* strain.

FIG. 3c is a photograph showing the result of two-dimensional chromatography analysis performed for comparison of polar lipids of the *Faecalibacterium cancerinhibens* strain CLCC1.

FIG. 3d is a photograph showing the result of two-dimensional chromatography analysis performed for comparison of polar lipids of the *Faecalibacterium prausnitzii* strain.

FIG. 3e is a graph of GC-MS analysis performing short-chain fatty acid analysis of *Faecalibacterium cancerinhibens* CLCC1.

FIG. 3f is a graph of GC-MS analysis performing short-chain fatty acid analysis of *Faecalibacterium prausnitzii.*

FIG. 4a to FIG. 4c are graphs showing the change in the tumor volume with time after inducing liver cancer to measure the inhibitory effect of cancer occurrence of *Faecalibacterium cancerinhibens* CLCC1 in mice induced with liver cancer according to Example 3-1. FIG. 4a is a dot graph showing each subject with gum, and FIG. 4b is a graph of comparing average values of the experimental group and the control group, and FIG. 4c is a polygonal graph showing the volume change of the tumor for each subject, respectively.

FIG. 5a to FIG. 5c are test results of the inhibitory effect of cancer occurrence of the *Faecalibacterium cancerinhibens* strain CLCC1 in a liver transplantation model according to Example 3-2 and dot graphs and polygonal graphs of each result. FIG. 5a represents the result of comparing tumor sizes directly, and FIG. 5b represents the result of representing the change in tumor sizes with a fold, and FIG. 5c represents a delta value.

FIG. 6a shows the change in tumor volume according to the dose of the *Faecalibacterium cancerinhibens* strain CLCC1 in mice subcutaneously transplanted with a human colorectal cancer cell line according to Example 4-1, and FIG. 6b shows the change in tumor weights, and FIG. 6c is the result of visually observing the change in tumor sizes.

FIG. 6d is the result of isolating and visually observing the tumor tissue in the negative control group (G1) and each experimental group (G2 to G4) after the end of the administration period of the *Faecalibacterium cancerinhibens* strain CLCC1 in mice subcutaneously transplanted with a human colorectal cancer cell line according to Example 4-1.

FIG. 6e is the result of performing H&E staining to confirm cell necrosis according to administration of the *Faecalibacterium cancerinhibens* strain CLCC1 in the tumor tissue of mice subcutaneously transplanted with a human colorectal cancer cell line.

FIG. 6f is the result of performing TUNEL staining to confirm apoptosis according to administration of the *Faecalibacterium cancerinhibens* strain CLCC1 in the tumor tissue of mice subcutaneously transplanted with a human colorectal cancer cell line.

FIG. 7 is the result of observing the change in tumor sizes according to administration of the *Faecalibacterium cancerinhibens* strain CLCC1 in the tumor tissue of mice subcutaneously transplanted with a mouse colorectal cancer cell line.

FIG. 8 is a diagram showing the body weight change of the animal model in an experiment of the colitis prevention and treatment effect using an animal model having Acute DSS-colitis according to Example 5.

FIG. 9 shows the result of measuring DAI (disease activity index) in an experiment of the colitis prevention and treatment effect using an animal model having Acute DSS-colitis according to Example 5.

FIG. 10 shows the length of the large intestine extracted after completing the experiment of the colitis prevention and treatment effect using an animal model having Acute DSS-colitis according to Example 5.

FIG. 11a to FIG. 11c are results of measuring the cytokine expression level in the experiment of the colitis prevention and treatment effect using an animal model having Acute DSS-colitis according to Example 5.

FIG. 12 is a photograph showing the pathological examination result of the large intestine tissue extracted after completing the experiment of the colitis prevention and treatment effect using an animal model having Acute DSS-colitis according to Example 5.

FIG. 13 shows the effect on human IL-8 of *Faecalibacterium cancerinhibens* in HT-29 cells analyzed by ELISA according to Example 7.

FIG. 14 shows the effect on mouse IL-6 of *Faecalibacterium cancerinhibens* in RAW 264.7 cells analyzed by ELISA according to Example 7.

FIG. 15 shows the effect on mouse TNF-$\alpha$ of *Faecalibacterium cancerinhibens* in RAW 264.7 cells analyzed by ELISA according to Example 7.

FIG. 16 shows the effect on human IL-10 of *Faecalibacterium cancerinhibens* in HPBMC analyzed according to Example 8.

FIG. 17 shows the effect on human IL-6 of *Faecalibacterium cancerinhibens* in HPBMC analyzed according to Example 8.

FIG. 18 is a graph showing the relative abundance of *Faecalibacterium cancerinhibens* in the intestinal microbial samples of the normal control group, ulcerative colitis (UC) and Crohn's disease patients according to one embodiment of the present invention.

[MODE FOR INVENTION]

The present invention will be described in more detail by the following illustrative examples, but it is not intended that the scope of the present invention is not limited by the following examples.

## Example 1. Isolation and identification of microorganism

### 1-1: Isolation of microorganism

*Faecalibacterium cancerinhibens* strain CLCC1 was isolated by diluting and applying feces of a healthy 20s adult male in a reinforced clostridial media (RCM) medium and then culturing at a room temperature of 37 °C under an anaerobic condition.

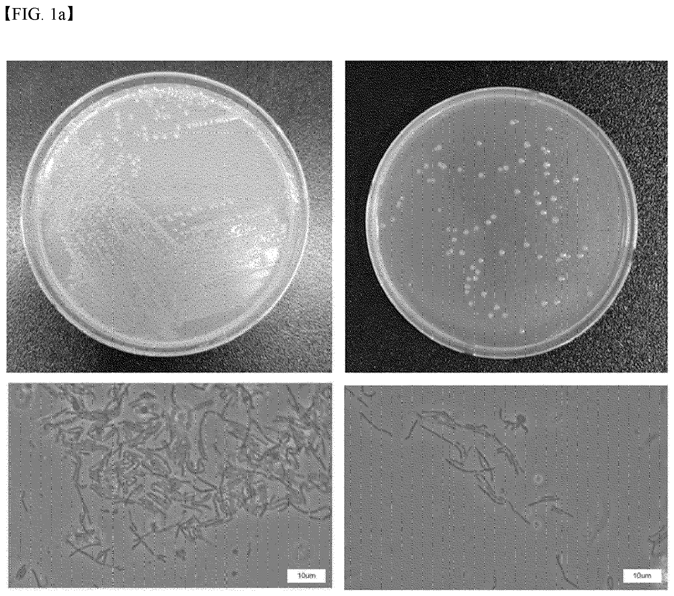

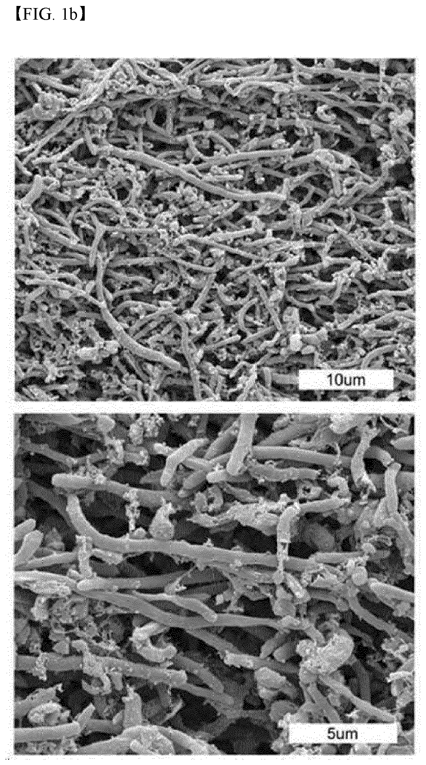

Specifically, *Faecalibacterium cancerinhibens* CLCC1 has a characteristic of forming colonies of 2 to 3mm at maximum and showing a long rod-shaped cell shape when observed with an optical microscope, when cultured in an RCM agar medium for 3 days. FIG. 1a showed the result of observing the colony form and the cell shape using an optical microscope, and FIG. 1b showed a photograph taking the cell shape with a scanning electron microscope.

### 1-2: Phylogenetic analysis using 16S rRNA analysis

*Faecalibacterium cancerinhibens* strain CLCC1 isolated by the method of Example 1 was cultured in an RCM liquid medium at a room temperature of 37 °C under an anaerobic culture condition for 1 day, and then centrifugation was performed to obtain a microorganism. Using FastDNA SPIN Kit for Soil (MP Biomedicals), the total genome was isolated from the obtained microorganism.

After producing a sequencing library using PacBio sequencing library kit, the total genome sequencing was performed using PacBio RS II platform, and using Whole Genome analysis pipeline of BIOiPLUG, our genome data analysis platform, the genome sequencing information was analyzed.

In Table 1 below, the genome characteristics of *Faecalibacterium cancerinhibens* strain CLCC1 were shown. It was shown that the total genome size of *Faecalibacterium cancerinhibens* strain CLCC1 was about 2.9Mbp, and the number of protein coding regions (CDS) was 2695, and the GC ratio (%) was 56.1%. It was shown that the total rRNA genes were 21 and the total tRNA genes were 69.

Phylogenetic analysis was performed by analyzing rRNA of *Faecalibacterium cancerinhibens* strain CLCC1 sequenced by the method. The 16S rRNA sequence of *Faecalibacterium cancerinhibens* strain CLCC1 was shown in SEQ ID NO: 1.

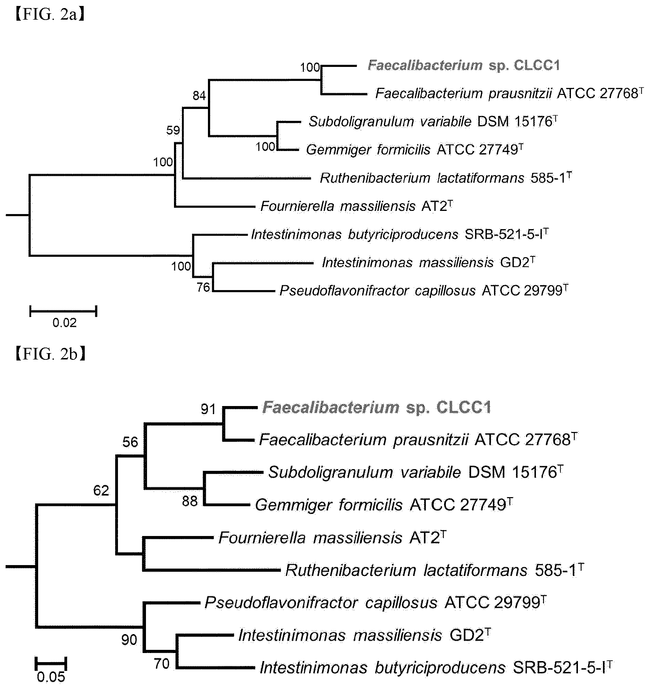

Specifically, the similarity analysis of 16S rRNA was performed using 16S-based identification for prokaryote pipeline of EzBioCloud database, and the phylogenetic analysis was conducted using MEGA program. In FIG. 2a, the phylogenetic position of *Faecalibacterium cancerinhibens* strain CLCC1 isolated by analyzing the 16S rRNA gene nucleotide sequence was shown.

### 1-3: Phylogenetic analysis using core genes

Phylogenetic analysis was performed through analysis between gene sequences using the genome sequence data obtained by the method of Example 2-1. Specifically, phylogenetic analysis was conducted using similarity of 92 genes (up-to-date bacterial core gene (UBCG)) which could be used as a taxonomic marker of bacteria. The 92 genes refer to genes provided by UBCG (up-to-date bacterial core gene) pipeline (Na, S. I., Kim, Y. O., Yoon, S. H., Ha, S. M.,

Baek, I. & Chun, J. (2018)). In FIG. 2b, a diagram showing phylogenetic positions with allied species obtained by analyzing 92 core genes was represented.

1-4: Relationship analysis using Average Nucleotide Identity (ANI)

Using the genome analysis data obtained by the method of Example 1-2, the relationship was analyzed by comparing ANI values of *Faecalibacterium cancerinhibens* strain CLCC1 and allied species.

Specifically, using Genome-based identification for prokaryote (TrueBAC ID) pipeline, an analysis platform provided by Chunlab, the genome similarity values were analyzed by comparing the total genome sequence of *Faecalibacterium cancerinhibens* strain CLCC1 and allied species.

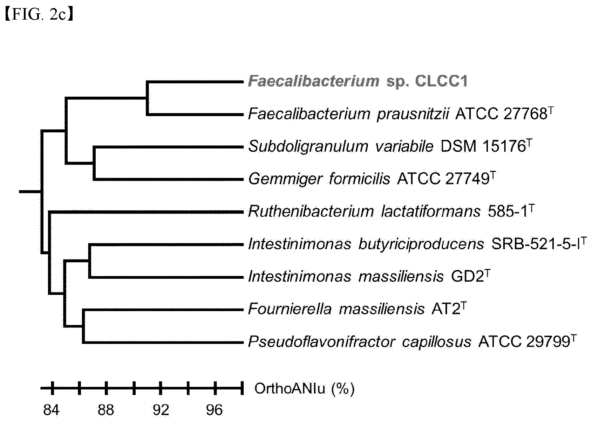

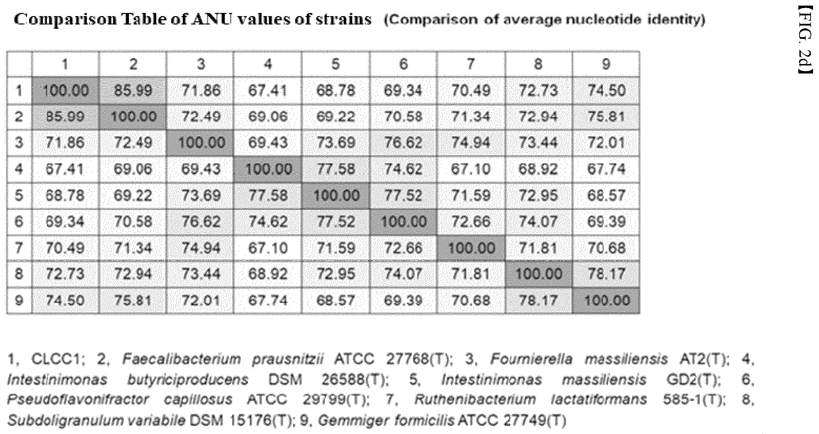

In FIG. 2c, the relationship diagram of *Faecalibacterium cancerinhibens* strain CLCC1 and allied species based on ANI values was shown. In FIG. 2d, the table of comparison of ANI values of *Faecalibacterium cancerinhibens* strain CLCC1 and allied species was shown. In each row and column, 1 means *Faecalibacterium cancerinhibens* strain CLCC1, and 2 means *Faecalibacterium prausnitzii* ATCC 27768(T), and 3 means *Fournierella massiliensis* AT2(T), and 4 means *Intestinimonas butyriciproducens* DSM 26588(T), and 5 means *Intestinimonas massiliensis* GD2(T), and 6 means *Pseudoflavonifractor capillosus* ATCC 29799(T), and 7 means *Ruthenibacterium lactatiformans* 585-1(T), and 8 means *Subdoligranulum variabile* DSM 15176(T), and 9 means *Gemmigerformicilis* ATCC 27749(T). (T) after each specific name means a type strain.

As the result of decoding the genome of *Faecalibacterium cancerinhibens* strain CLCC1 and comparing with allied species, the strain CLCC1 showed an average nucleotide identity (ANI) value of 85.99% at a genome level with *Faecalibacterium prausnitzil* which is the closest allied species at the total genome level, and it can be found that it is a novel species not isolated and reported conventionally. The *Faecalibacterium* spp. strain identified by the above method was named *Faecalibacterium cancerinhibens* CLCC1.

**Example 2. Analysis of physiochemical characteristics of microorganism**

2-1: Analysis of fatty acid content

To analyze the molecular biological characteristics *of Faecalibacterium cancerinhibens* strain CLCC1, the fatty acid composition of *Faecalibacterium cancerinhibens* strain CLCC1 and *Faecalibacterium prausnitzii* was compared.

Specifically, *Faecalibacterium cancerinhibens* strain CLCC1 and *Faecalibacterium prausnitzii* strain were cultured in an RCM medium at a temperature of 37°C under an anaerobic condition, respectively, and then using microbial cells of 40mg, fatty acids were obtained according to the method of Miller (Miller, L. T. (1982) J. Clin. Microbiol. 18, 861-867).

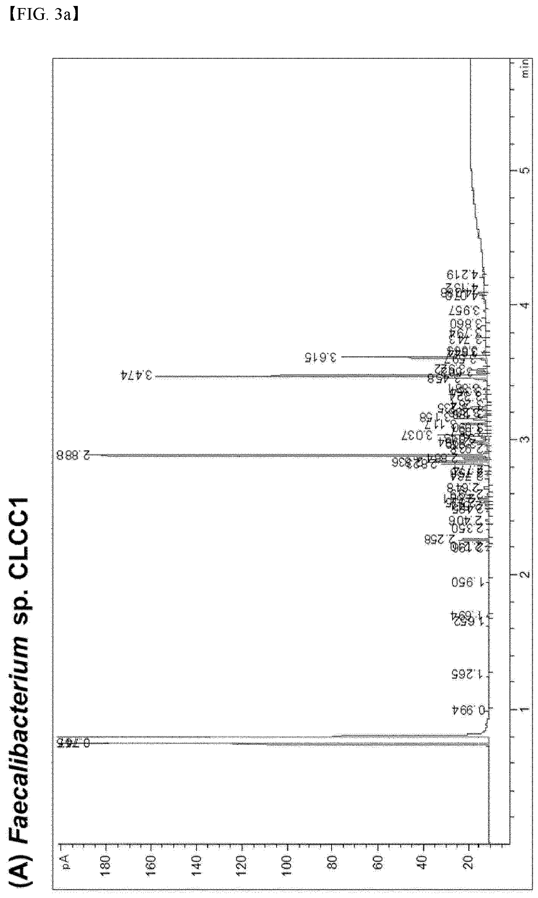

In analysis of the fatty acids extracted, Agilent technologies 6890 Gas chromatography was used, and as a separation column, A30m X 0.320mm X 0.25μm Crosslinked Methyl siloxane column (HP-1) was used. In FIG. 3a, a graph of the result of gas chromatography analysis of comparing the fatty acid composition of *Faecalibacterium cancerinhibens* strain CLCC1 and *Faecalibacterium prausnitzii* strain (ATCC 27768) was shown. In the graph of FIG. 3a, the names of fatty acids that each peak means in the graph and their values (%, w/v) were shown in Table 1 below.

[Table 1]

| Fatty acid type | Content in CLCC1 (%) | Content in ATCC 27768 (%) |
|---|---|---|
| 16:00 | 32.42 | 42.28 |
| 18:1 7 | 28.15 | 14.88 |
| 17:0 2OH | 12.51 | 8.09 |
| 16:1 ω7c/16:1 ω6c | 5.22 | 6.04 |
| 16:1 ω9c | 3.89 | 1.57 |
| 14:00 | 2.44 | 14.93 |
| 17:0 anteiso | 2.42 | 0.99 |
| 16:1 ω5c | 2.17 | 0.83 |
| 18:1 ω9c | 1.88 | 0.86 |
| 18:00 | 1.59 | 2.17 |

(continued)

| Fatty acid type | Content in CLCC1 (%) | Content in ATCC 27768 (%) |
| --- | --- | --- |
| 16:1 2OH | 1.41 | 0.51 |
| 20:1 ω7c | 0.84 | 0.43 |
| 18:1 ω5c | 0.81 | 0.32 |
| 15:0 2OH | 0.75 | 0.5 |
| 18:0 10-methyl, TBSA | 0.45 | 0.21 |
| 15:1 ω8c | 0.44 | - |
| 15:1 iso H/13:0 3OH | 0.35 | 1.44 |
| 16:0 3OH | 0.32 | 0.11 |
| 15:0 anteiso | 0.21 | 0.2 |
| 12:00 | 0.2 | 1.59 |
| 15:0 iso 3OH | 0.17 | 0.49 |
| 17:00 | 0.17 | 0.2 |
| 19:0 cyclo ω10c/19ω6 | 0.16 | - |
| 20:00 | 0.14 | 0.1 |
| 13:1 at 12-13 | 0.13 | 0.35 |
| 10:00 | 0.13 | 0.2 |
| 20:1 ω9c | 0.13 | - |
| 17:0 iso | 0.12 | 0.17 |
| 14:0 3OH/16: 1 iso I | 0.12 | - |
| 15:0 3OH | 0.11 | - |
| 16:0 iso | 0.1 | - |
| 16:0 iso 3OH | 0.05 | - |
| 17:0 iso 3OH | - | 0.54 |

As shown in the Table 1, 15:1 ω8c fatty acid, 19:0 cyclo ω10c/19ω6 fatty acid, 20:1 ω9c fatty acid, 14:0 3OH/16: 1 iso I fatty acid, 15:0 3OH fatty acid, 16:0 iso fatty acid and 16:0 iso 3OH fatty acid were detected only in strain CLCC1, and 17:0 iso 3OH fatty acid was detected only in *Faecalibacteriumprausnitzii* ATCC 27768.

As the result of comparison analysis of the molecular biological characteristics, it could be confirmed that *Faecalibacterium cancerinhibens* strain CLCC1 was a different species from *Faecalibacterium prausnitzii* which is a known species included in *Faecalibacterium* spp. When comparing the fatty acid composition of two microorganisms, it could be found that there was a difference in the composition of major fatty acids between two microorganisms, and the corresponding result was shown in FIG. 3a and Table 2.

2-2: Polar lipid analysis

To compare the polar lipid composition, thin layer chromatography (TLC) analysis was performed.

Specifically, the polar lipid was extracted from a microorganism lyophilized sample of 50 mg by the method of Minikin (Minikin, D.E., et al. (1984). J Microbial Meth 2, 233-241.). In addition, the primary development was performed in a solvent having a ratio of chloroform, methanol and water of 65:25:3.8(v/v) (chloroform: methanol: water = 65:25:3.8(v/v)). Then, secondary development was formed in a solvent having a ratio of chloroform, methanol, acetate and water of 40:7.5:6:1.8(v/v) (chloroform:methanol:acetic acid:water = 40:7.5:6:1.8(v/v)). After the secondary development, it was stained with 5%(w/v) ethanolic molybdatophosphoric acid.

In addition, when comparing the composition of the polar lipid, it was confirmed that *Faecalibacterium cancerinhibens* strain CLCC1 and *Faecalibacterium prausnitzii* had one phosphatidylglycerol (PG), two unknown unidentified phospholipids (PL1, PL2), and a plurality of unidentified lipids (L). In addition, it was confirmed that strain CLCC1 had unidentified phospholipids (PL3) not present in *Faecalibacterium prausnitzii* additionally, and the corresponding result was shown in FIG. 3b. As could be confirmed from the result, it can be found that the polar lipid production patterns of *Faecalibacterium cancerinhibens* CLCC1 according to the present invention and Faecalibacterium prausnitzii are different.

2-3: Short chain fatty acid (SCFA) analysis

To examine the difference of short chain fatty acid production patterns of *Faecalibacterium prausnitzii* and *Faecalibacterium cancerinhibens* CLCC1 microorganism, by requesting analysis to Korea Research Institute of Bio medical Science, the analysis of the short chain fatty acid distribution was performed.

Specifically, after culturing *Faecalibacterium prausnitzii* and *Faecalibacterium cancerinhibens* strain CLCC1 of the present invention in an RCM medium for 16 hours, cells were precipitated by centrifugation (4°C, 4,000 rpm, 10min) to collect the supernatant. The collected supernatant was refrigerated after filtering with a 0.22um filter. A GC-MS sample for SCFA analysis from the supernatant was prepared using the method of Takeshi Furuhashi (Takeshi Furuhashi, et al., Analytical Biochemistry, Volume 543, 2018, Pages 51-54), and 6890 series equipment of Agilent was used for the short chain fatty acid analysis using GC-MS.

As the result of comparison analysis of short chain fatty acids (SCFA) present in the culture supernatant of *Faecalibacterium cancerinhibens* CLCC1 and *Faecalibacterium prausnitzii,* other known species of *Faecalibacterium* spp., in both samples, as SCFA, only butyric acid (butanoic acid) was detected. In addition, butyric acid (butanoic acid) at a content 7.8 times higher was detected in the *Faecalibacterium cancerinhibens* CLCC1 sample, compared to the *Faecalibacterium prausnitzii* sample. In FIGs. 3c to 3d, the graph of the GC-MS analysis was represented.

Furthermore, when looking at the ratio of butyric acid in each sample, 91.64% of the culture supernatant of *Faecalibacterium cancerinhibens* CLCC1 was butyric acid, and 76.40% of the culture supernatant of *Faecalibacterium prausnitzii* was butyric acid. The result of the short chain fatty acid analysis of *Faecalibacterium cancerinhibens* strain CLCC1 was shown in Table 2, and the result of the short chain fatty acid analysis of *Faecalibacterium prausnitzii* was shown in Table 3. Other substances indicated in Table 2 to Table 3 below were detected in the substance library database, but they were classified as noise signals that could not accurately identify the substances because of their very low matching rates (quality). In Table 2 and Table 3 below, RT means a retention time.

[Table 2]

| Peak | RT | Library/ID | Matching rate (Quality) | Area | Ratio (% of total, %) |
|---|---|---|---|---|---|
| 1 | 2.6965 | Oxirane | 58 | 606,058 | 3.132 |
| 2 | 3.1915 | Butanoic acid (CAS); n-Butyric acid | 94 | 17,732,33 7 | 91.635 |
| 3 | 3.5174 | Ethane, methoxy- (CAS); Methane | 50 | 497,085 | 2.569 |
| 4 | 4.1933 | 2-Pentanone, 4-hydroxy-4-methyl- (CAS) | 43 | 515,638 | 2.665 |

[Table 3]

| Peak | RT | Library/ID | Matching rate (Quality) | Area | Ratio (% of Total, %) |
|---|---|---|---|---|---|
| 1 | 2.6908 | 2-Decanone (CAS) | 38 | 338,372 | 11.491 |
| 2 | 3.0167 | Butanoic acid (CAS); n-Butyric acid | 93 | 2,249,717 | 76.401 |
| 3 | 4.2057 | 2-Pentanone, 4-hydroxy-4-methyl- (CAS) | 47 | 356,542 | 12.108 |

It can be predicted that *Faecalibacterium cancerinhibens* strain CLCC1 of the present invention exhibits an excellent anti-cancer activity, due to higher production of butyric acid among short chain fatty acids, compared to *Faecalibacterium prausnitzii.*

**Example 3. Efficacy evaluation for liver cancer**

3-1: Test of inhibitory efficacy of cancer occurrence in subcutaneous tumor model

To confirm the inhibiting ability of cancer occurrence of *Faecalibacterium cancerinhibens* strain CLCC1, the inhibitory efficacy of cancer occurrence was tested in a subcutaneous tumor model using a mouse liver cancer cell line. In 10 6-week-age male C57BL/6 mice, through a subcutaneous tumor transplantation experiment of a liver cancer cell line (Hep55.1c), liver cancer was caused, and during a process of liver cancer occurrence for 55 days, the effect of oral administration of *Faecalibacterium cancerinhibens* strain CLCC1 on the growth of liver cancer cells was examined. The liver cancer cell line Hep55.1c was mouse-derived hepatocellular carcinoma (hepatoma).

Specifically, *Faecalibacterium cancerinhibens* CLCC1 was orally administered 4 days before the mouse liver cancer cell line was transplanted into the mouse subcutaneous tissue. At Day 4 after oral administration, the mouse liver cancer cell line (Hep55.1c) was transplanted into the subcutaneous tissue of the right flank to make $2.0 \times 10^6$ cells.

For the subcutaneous tumor model using the mouse liver cancer cell line, *Faecalibacterium cancerinhibens* strain CLCC1 was orally administered once a day, 5 times a week, for 55 days. Then, from the day of transplantation (Day 0) to Day 55, the size of the liver cancer tumor (tumor volume) was measured using Magnetic Resonance (MR) imaging at a 7-day interval.

*Faecalibacterium cancerinhibens* strain CLCC1 was cultured in an RCM medium, and then concentrated and stored frozen using a PBS buffer and glycerol to a final glycerol concentration of 15%(v/v). In addition, after thawing immediately before administration to mice, 200ul ($2 \times 10^8$ cells) per animal was orally administered. As a control group, 10 mice not administered with strain CLCC1 were used. For the control group, 200ul each of PBS and glycerol (15%, v/v) solution containing no strain was orally administered.

In Table 4 and Table 5 below, the result of the tumor size of the control group and test group in the test of the inhibitory efficacy of liver cancer occurrence of the isolated strain in the subcutaneous tumor model was shown. Table 5 is the result of the tumor volume change for the control group, and Table 6 is the result of the tumor size (volume) change of the experimental group administered with *Faecalibacterium cancerinhibens* strain CLCC 1. In the tables, S.D means standard deviation.

【Table 4】

| Day | | | | | | Control | | | | | | | |
|-----|------|------|------|------|------|------|------|------|------|------|------|------|------|
| | 1 | 2 | 3 | 4 | 5 | 6 | 7 | 8 | 9 | 10 | Mean | S.D. |
| 0 | 0.0 | 0.0 | 0.0 | 0.0 | 0.0 | 0.0 | 0.0 | 0.0 | 0.0 | 0.0 | 0.0 | 0.00 |
| 4 | 31.2 | 1.3 | 5.8 | 14.3 | 5.5 | 2.9 | 0.8 | 2.6 | 5.0 | 20.0 | 8.9 | 9.90 |
| 7 | 47.4 | 18.2 | 27.1 | 13.2 | 12.6 | 16.2 | 13.8 | 13.2 | 19.3 | 23.9 | 20.5 | 10.64 |
| 10 | 132.0 | 55.4 | 74.7 | 51.4 | 79.7 | 81.7 | 70.4 | 50.0 | 76.5 | 101.1 | 77.3 | 24.80 |
| 13 | 132.3 | 81.9 | 100.2 | 115.5 | 147.2 | 86.8 | 73.5 | 55.6 | 99.0 | 184.3 | 107.6 | 38.31 |
| 17 | 160.0 | 115.9 | 120.0 | 99.4 | 144.7 | 126.8 | 101.3 | 124.7 | 102.6 | 182.9 | 127.8 | 27.29 |
| 20 | 236.7 | 223.2 | 121.8 | 173.4 | 206.3 | 137.6 | 136.4 | 119.0 | 165.8 | 348.9 | 186.9 | 70.61 |
| 24 | 282.5 | 261.1 | 124.6 | 250.0 | 303.4 | 249.0 | 185.1 | 160.0 | 203.7 | 388.3 | 240.8 | 76.37 |
| 27 | 547.9 | 393.6 | 205.3 | 302.7 | 527.5 | 323.3 | 262.7 | 252.5 | 294.1 | 469.5 | 357.9 | 120.33 |
| 31 | 425.0 | 513.5 | 323.3 | 338.6 | 488.8 | 341.2 | 323.0 | 307.8 | 312.7 | 554.8 | 392.9 | 94.30 |
| 34 | 571.6 | 570.2 | 402.5 | 489.1 | 611.4 | 387.2 | 366.2 | 410.1 | 380.5 | 728.3 | 491.7 | 123.16 |
| 38 | 562.0 | 586.1 | 502.6 | 517.2 | 819.3 | 557.2 | 529.1 | 509.2 | 493.4 | 819.3 | 589.5 | 124.50 |
| 41 | 563.2 | 902.2 | 574.0 | 673.2 | 761.5 | 506.4 | 626.6 | 659.6 | 604.4 | 1008.7 | 688.0 | 158.98 |
| 45 | 815.8 | 1009.7 | 883.2 | 759.6 | 1222.4 | 727.7 | 806.3 | 753.0 | 656.1 | 1101.4 | 873.5 | 181.41 |
| 48 | 904.1 | 784.4 | 1035.6 | 772.8 | 1074.4 | 735.2 | 726.5 | 802.0 | 701.6 | 1306.9 | 884.4 | 196.55 |
| 52 | 1039.7 | 1413.8 | 1031.2 | 1128.0 | 1146.8 | 940.3 | 981.9 | 939.7 | 807.5 | 1623.9 | 1105.3 | 243.74 |
| 55 | 1098.8 | 2037.5 | 1201.0 | 1327.7 | 1636.2 | 1076.8 | 993.3 | 961.8 | 930.0 | 1813.3 | 1307.6 | 389.62 |

【Table 5】

| Day | 1 | 2 | 3 | 4 | 5 | 6 | 7 | 8 | 9 | 10 | Mean | S.D. | p-value |
|---|---|---|---|---|---|---|---|---|---|---|---|---|---|
| | | | | | CLCC1 | | | | | | | | |
| 0 | 0.0 | 0.0 | 0.0 | 0.0 | 0.0 | 0.0 | 0.0 | 0.0 | 0.0 | 0.0 | 0.0 | 0.00 | |
| 4 | 5.5 | 4.3 | 0.9 | 0.0 | 2.1 | 4.3 | 1.8 | 0.0 | 0.8 | 2.6 | 2.2 | 1.93 | |
| 7 | 24.8 | 16.0 | 10.2 | 3.6 | 7.7 | 19.7 | 15.4 | 14.0 | 1.8 | 9.0 | 12.2 | 7.14 | |
| 10 | 86.5 | 59.6 | 52.3 | 19.5 | 57.5 | 64.4 | 72.1 | 60.7 | 12.7 | 60.8 | 54.6 | 22.42 | |
| 13 | 96.6 | 74.9 | 89.9 | 65.2 | 63.6 | 101.3 | 94.6 | 119.4 | 48.9 | 78.3 | 83.3 | 21.01 | 0.0949 |
| 17 | 121.6 | 99.8 | 112.6 | 76.9 | 103.9 | 122.5 | 89.3 | 116.4 | 112.9 | 73.1 | 102.9 | 17.87 | 0.0266 |
| 20 | 164.0 | 169.8 | 160.7 | 63.1 | 121.3 | 190.0 | 83.9 | 135.3 | 63.9 | 101.1 | 125.3 | 45.90 | 0.0328 |
| 24 | 226.0 | 173.4 | 165.6 | 101.3 | 125.4 | 302.6 | 150.2 | 147.3 | 114.4 | 131.4 | 163.8 | 60.16 | 0.0221 |
| 27 | 245.2 | 290.1 | 214.1 | 101.3 | 149.3 | 317.7 | 222.5 | 208.4 | 145.1 | 113.3 | 200.7 | 72.73 | 0.0024 |
| 31 | 315.9 | 412.5 | 363.9 | 216.1 | 229.7 | 436.9 | 218.3 | 371.0 | 245.6 | 280.6 | 309.0 | 82.98 | 0.0491 |
| 34 | 388.7 | 326.8 | 338.0 | 137.4 | 238.1 | 547.0 | 301.6 | 397.3 | 288.7 | 246.6 | 321.0 | 110.22 | 0.0043 |
| 38 | 516.4 | 353.8 | 470.6 | 508.0 | 206.2 | 822.4 | 336.8 | 436.1 | 308.2 | 311.8 | 427.0 | 170.79 | 0.0257 |
| 41 | 688.5 | 494.5 | 448.9 | 470.2 | 382.2 | 1000.9 | 414.0 | 632.5 | 461.5 | 430.2 | 542.3 | 187.73 | 0.0775 |
| 45 | 618.5 | 466.7 | 538.2 | 655.2 | 468.3 | 1058.5 | 462.1 | 712.4 | 246.4 | 484.0 | 571.0 | 214.54 | 0.0032 |
| 48 | 919.4 | 485.3 | 717.5 | 970.2 | 393.3 | 1233.6 | 558.0 | 682.7 | 262.8 | 556.6 | 677.9 | 293.19 | 0.0809 |
| 52 | 936.4 | 441.7 | 789.0 | 1347.2 | 596.2 | 1265.5 | 623.3 | 1063.4 | 548.1 | 620.4 | 823.1 | 315.46 | 0.0381 |
| 55 | 1049.2 | 494.9 | 941.8 | 1369.1 | 595.1 | 1068.7 | 573.9 | 718.0 | 384.5 | 472.9 | 766.8 | 323.14 | 0.0033 |

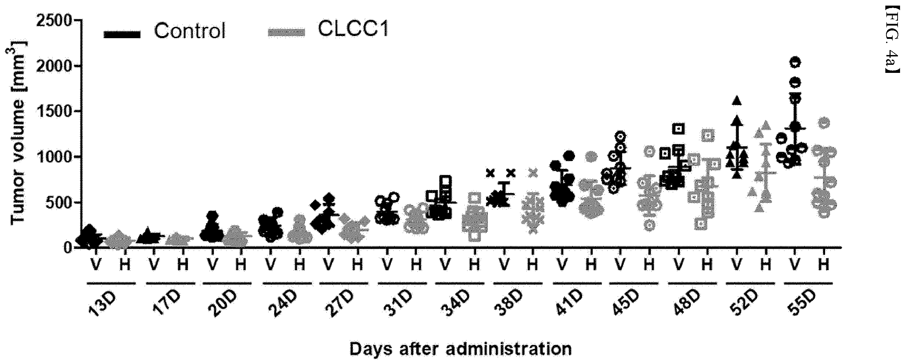

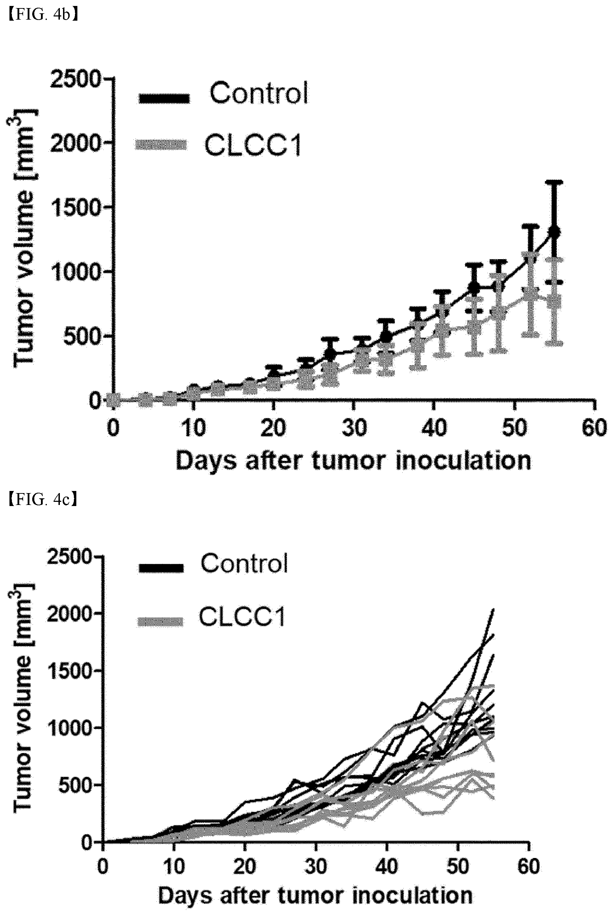

In FIG. 4, after inducing cancer in mice, the tumor volume according to the time change was shown as a graph, and in the Tables 5 to 6, the change in the tumor volume was shown as numerical values.

As the experimental result, it was confirmed that the liver cancer occurrence and progress were statistically significantly inhibited in the experimental group orally administered with CLCC1, compared to the control group. The tumor volume was averagely about from about 19.5% to 75.3%, on average, 32.9% lower, in the experimental group orally administered with CLCC1, compared to the control group, and thereby it was confirmed that in the experimental group administered with *Faecalibacterium cancerinhibens* strain CLCC1, the liver cancer occurrence was delayed, and the tumor growth progress rate was inhibited. At Day 4 which is the first day of measuring the tumor size after surgery, it can be confirmed that there is an inhibitory effect of cancer occurrence, as the tumor size is definitely small in the experimental group administered with *Faecalibacterium cancerinhibens* strain CLCC1, compared to the control group. Thus, it can be found that orally administered strain CLCC1 has an activity to inhibit occurrence and progress of liver cancer.

3-2: Test of inhibitory efficacy of cancer occurrence in liver transplantation model

To examine the effect of *Faecalibacterium cancerinhibens* strain CLCC1 on the growth of liver cancer cells, in 4 8-week-age male C57BL/6 mice, through a liver transplantation experiment of a liver cancer cell line, liver cancer was caused, and during a process of liver cancer occurrence for 39 days, the effect of oral administration of *Faecalibacterium cancerinhibens* strain CLCC1 on the growth of liver cancer cells was examined.

Specifically, as the liver cancer cell line, Hep55.1c was used, and the epigastrium of the 8-week-old male C57BL/6 mice was opened, and the liver cancer cell line was transplanted into the left lobe of liver so as to be $5.0 \times 10^5$ cells. After a recovery period of 4 days after transplanting the tumor cell line into the liver, the tumor size was confirmed by MRI scanning, and 4 mice in which tumor was well formed were selected and used for a subsequent experiment.

For 39 days from the day of transplantation (Day 0), strain CLCC1 was prepared by the same method as Example 4, and orally administered to 4 mice that completed the transplantation operation, and strain CLCC1 was not administered to 4 control mice. For the control group, 200ul each of PBS and glycerol (15%, v/v) solution containing no strain was orally administered. Thereafter, the tumor size and relative size and relative value of growth were periodically measured.

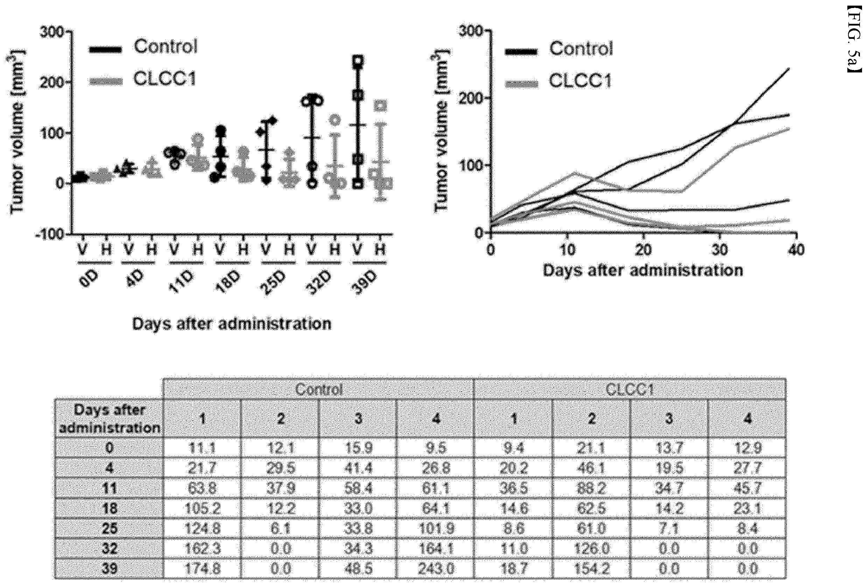

The tumor size was measured by magnetic resonance (MR) imaging at a 7-day interval, and the relative value of the tumor size was drawn by calculating a relative size to the tumor size at the day of transplantation. In FIG. 5a to FIG. 5c, the test result and the graph of the inhibitory effect of cancer occurrence of strain CLCC1 in the liver transplantation model were shown.

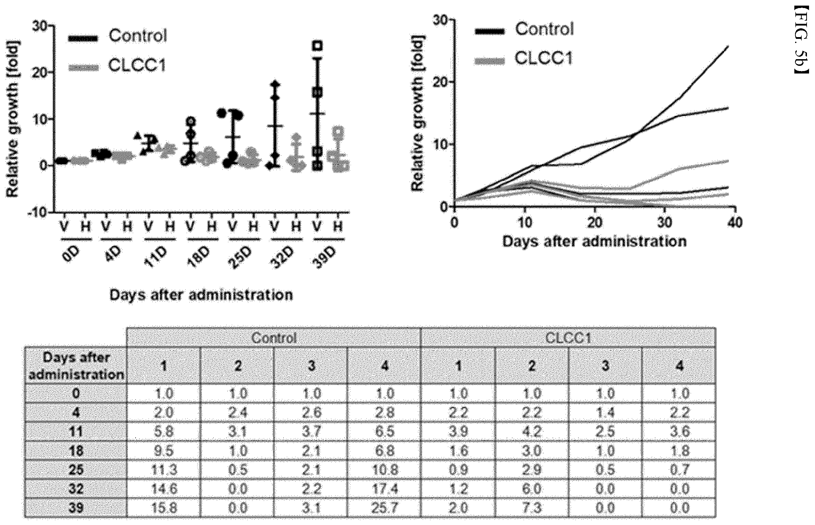

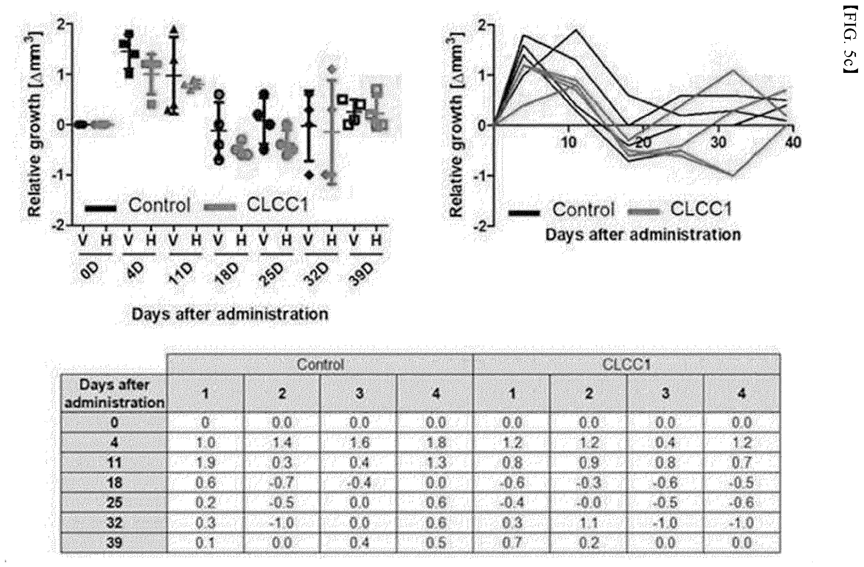

FIG. 5a is a dot graph and a polygonal graph which directly compare the tumor size, and FIG. 5b is dot and polygonal graphs representing the change in the tumor size by fold, and FIG. 5c is dot and polygonal graphs representing the change amount of the tumor size (difference between the tumor size on the day of measurement and the tumor size on the day of previous measurement).

As a result, it was confirmed that the liver cancer occurrence and progress were statistically significantly inhibited in the experimental group orally administered with CLCC1, compared to the control group not administered with the isolated line. The result for inhibition of liver cancer occurrence and data of the change in the tumor size were shown in FIGs. 5a to 5c.

The relative figure table (Relative growth; Fold) of the tumor size was shown in Table. 7, and the relative figure table (Relative growth; Delta) of the tumor growth is shown in Table 7.

[Table 6]

| Days after administration | Control | | | | CLCCI | | | |
|---|---|---|---|---|---|---|---|---|
| | 1 | 2 | 3 | 4 | 1 | 2 | 3 | 4 |
| 0 | 1.0 | 1.0 | 1.0 | 1.0 | 1.0 | 1.0 | 1.0 | 1.0 |
| 4 | 2.0 | 2.4 | 2.6 | 2.8 | 2.2 | 22 | 14 | 2.2 |
| 11 | 5.8 | 31 | 31 | 6.5 | 39 | 42 | 2.5 | 3.6 |
| 18 | 9.5 | 1.0 | 2.1 | 6.8 | 1.6 | 3.0 | 1.0 | 1.8 |
| 25 | 11.3 | 0.5 | 2.1 | 10.8 | 0.9 | 2.9 | 0.5 | 0.7 |
| 32 | 14.6 | 0.0 | 2.2 | 17.4 | 1.2 | 6.0 | 0.0 | 0.0 |
| 39 | 15.8 | 0.0 | 3.1 | 25.7 | 2.0 | 7.3 | 0.0 | 0.0 |

[Table 7]

| Days after administration | Control | | | | CLCCI | | | |
|---|---|---|---|---|---|---|---|---|
| | 1 | 2 | 3 | 4 | 1 | 2 | 3 | 4 |
| 0 | 0 | 0.0 | 0.0 | 0.0 | 0.0 | 0.0 | 0.0 | 0.0 |
| 4 | 1.0 | 1.4 | 1.6 | 1.8 | 1.2 | 1.2 | 0.4 | 1.2 |
| 11 | 1.9 | 0.3 | 04 | 1.3 | 0.8 | 0.9 | 0.8 | 0.7 |
| 18 | 0.6 | -0.7 | -0.4 | 0.0 | -0.6 | -0.3 | -0.6 | -05 |
| 25 | 02 | -0.5 | 0.0 | 0.6 | -0.4 | -0.0 | -0.5 | -0.6 |
| 32 | 0.3 | -1.0 | 0.0 | 0.6 | 0.3 | 1.1 | -1.0 | -1.0 |
| 39 | 0.1 | 0.0 | 0.4 | 0.5 | 0.7 | 0.2 | 0.0 | 0.0 |

As can be confirmed in Table 6 and Table 7 and FIGs. 5a to 5b, it can be confirmed that the tumor growth of the mice orally administered with strain CLCC1 was remarkably inhibited, compared to the control group. Thus, it can be confirmed that *Faecalibacterium cancerinhibens* strain CLCC1 has an excellent inhibitory ability of tumor growth.

**Example 4. Efficacy evaluation for colorectal cancer**

**4-1. Confirmation of anti-cancer cancer in subcutaneous tumor model**

To confirm the inhibitory ability of cancer occurrence of *Faecalibacterium cancerinhibens* strain CLCC1, the inhibitory efficacy of cancer occurrence in a subcutaneous tumor model using a human colorectal cancer cell line was tested. As the colorectal cancer cell line, HCT-116 cells were used, and as the mouse, nude mice (CAnN.Cg-Foxn1nu/CrlOri, SPF) were used. The HCT-116 is a cancer cell line corresponding to human-derived colorectal carcinoma.

Specifically, the colorectal cancer cell line HCT-116 (2.Sx10' cells/mL) was administered to the mice that had undergone a one-week purification period and transplanted by administering 0.2 mL/head subcutaneously to the right back of mice using a disposable syringe. When the size of cancer cells grew to about 85~119mm$^3$ after cancer cell transplantation, 10 mice per group were classified into a negative control group not administered with CLCC1 and 3 experimental groups administered with strain CLCC1 by concentration. Specifically, the experimental group was divided into 3 groups of low, medium and high according to the concentration of strain CLCC1, and F. *cancerinhibens* strain CLCC1 was administered at a concentration of $2\times10^6$ cells/head for the low concentration, $2\times10^7$ cells/head for the medium concentration, and $2\times10^8$ cells/head for the high concentration. The administration of the strain was oral administration once a day for 36 days with a syringe. For the negative control group, only an excipient (PBS solution comprising glycerol 15%(v/v)) was administered. In Table 8 below, the information of the negative control group and each experimental group was shown.

[Table 8]

| Mark | CLCC1 dose | Administration solution amount | Description | Number of subj ects (number of animals) |
|---|---|---|---|---|
| G1 | 0 | 0.2mL/head | Negative control group | 10 |
| G2 | $2\times10^6$(cells/head) | 0.2mL/head | Low concentration experimental group | 10 |
| G3 | $2\times10^7$(cells/head) | 0.2mL/head | Medium concentration experimental group | 10 |
| G4 | $2\times10^8$(cells/head) | 0.2mL/head | High concentration experimental group | 10 |

**(1) Tumor size change and growth inhibition rate**

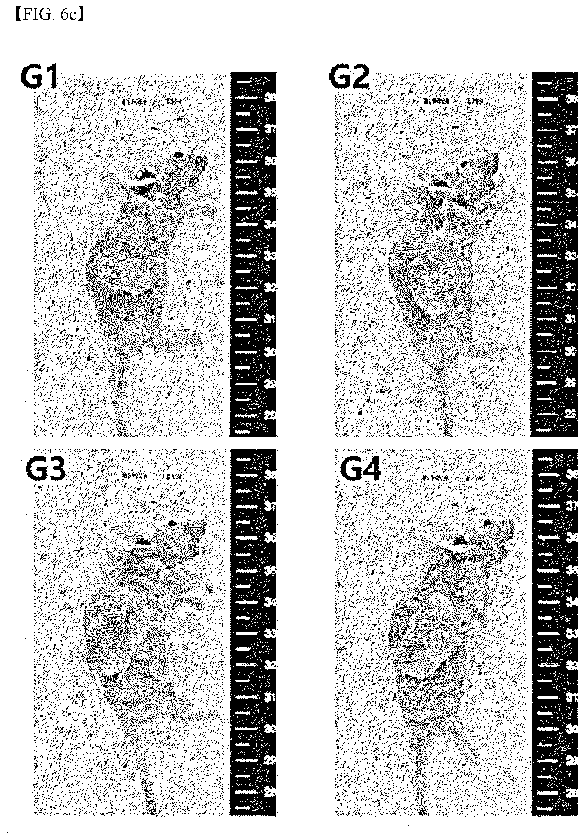

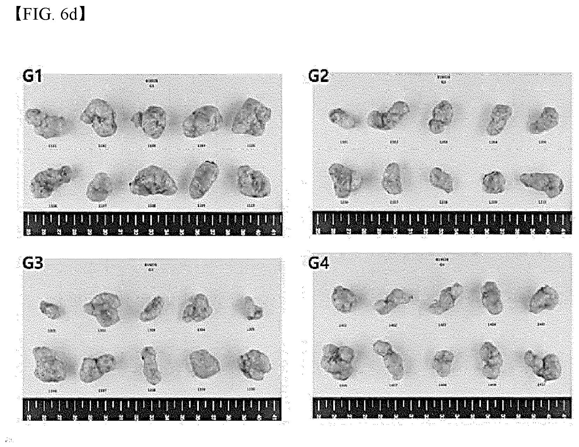

To confirm the anti-colorectal cancer effect of F. *cancerinhibens* strain CLCC1, the change in the tumor size was confirmed. Specifically, once a day after starting the test of strain administration, general symptoms such as appearance, behavior, excretion and the like were observed, and the body weight was measured once a week, and the tumor size was measured, and the volume was calculated twice a week. In FIGs. 6c and 6d, the result of visually observing the tumor size was shown.

The tumor volume (Tv) was calculated by the method of Equation 3 below by measuring the perpendicular width (W) and maximum length (L) of tumor, respectively.

[Equation 3]

$$Tv\ (mm^3) = L\ (mm)\ x\ W^2\ (mm^2)\ x\ 1/2$$

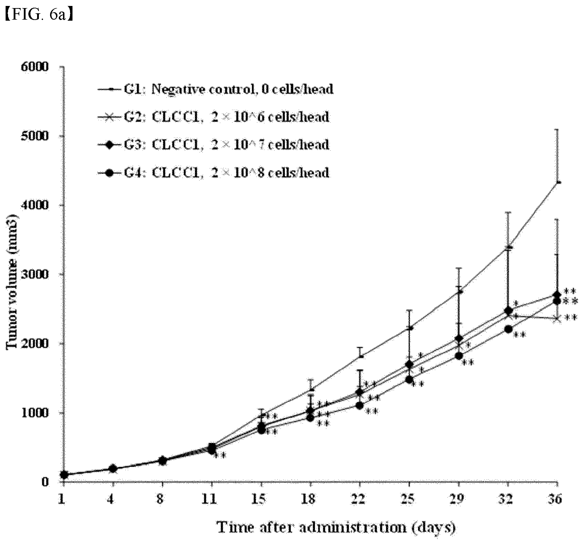

The result of confirming the change in the tumor volume throughout 36 days was shown in Table 9 and FIG. 6a below. It was confirmed that the tumor size was statistically significantly reduced in all the experimental groups administered with strain CLCC1, compared to the negative control group (G1). In Table 9 below, Mean refers to an average volume and S.D. means standard deviation.

[Table 9]

| Group/ Dose (cells/head) | | Tumor volume (mm³) | | | | | | | | | | |
|---|---|---|---|---|---|---|---|---|---|---|---|---|
| | | Time after administration (days) | | | | | | | | | | |
| | | 1 | 4 | 8 | 11 | 15 | 18 | 22 | 25 | 29 | 32 | 36 |
| G1 | Mean | 105 | 186 | 316 | 520 | 968 | 1331 | 1809 | 2228 | 2748 | 3399 | 4330 |
| 0 | S.D. | 10 | 15 | 26 | 39 | 87 | 145 | 136 | 251 | 347 | 497 | 763 |
| G2 | Mean | 105 | 187 | 304 | 479 | 802 | 1029 | 1269 | 1629 | 1974 | 2408 | 2651 |
| $2\times10^6$ | S.D. | 9 | 17 | 30 | 56 | 127 | 238 | 347 | 572 | 744 | 944 | 931 |
| | | | | | | ** | ** | ** | * | * | * | ** |
| G3 | Mean | 105 | 193 | 312 | 497 | 812 | 1032 | 1306 | 1705 | 2078 | 2481 | 2875 |
| $2\times10^7$ | S.D. | 9 | 20 | 32 | 48 | 143 | 216 | 303 | 544 | 750 | 921 | 1007 |
| | | | | | | ** | ** | ** | * | | * | ** |

(continued)

| Group/ Dose (cells/head) | | Tumor volume (mm$^3$) | | | | | | | | | | |
|---|---|---|---|---|---|---|---|---|---|---|---|---|
| | | Time after administration (days) | | | | | | | | | | |
| | | 1 | 4 | 8 | 11 | 15 | 18 | 22 | 25 | 29 | 32 | 36 |
| G4 | Mean | 105 | 191 | 303 | 452 | 754 | 926 | 1112 | 1487 | 1821 | 2210 | 2621 |
| 2×10$^8$ | S.D. | 9 | 16 | 40 | 53 | 104 | 201 | 163 | 317 | 471 | 607 | 701 |
| | | | | | ** | ** | ** | ** | ** | ** | ** | ** |

\* p<0.05, Significant difference from the negative control group (G1) by Dunnett's t-test.
\*\* p<0.01, Significant difference from the negative control group (G1) by Dunnett's t-test.

Specifically, the average tumor size at the day (Day 1) of staring administration of the microorganism was same as 105mm$^3$ in all the 4 groups, but in 36 days, the average tumor volume was shown as 4330mm$^3$ in the negative control group, and on the other hand, the volume of 3000mm$^3$ or less was shown in all the experimental groups administered with strain CLCC1, and therefore, it was confirmed that it had the tumor volume of about 60 to 67%, compared to the control group, and thereby, the inhibitory effect of the colorectal cancer growth of the *Faecalibacterium cancerinhibens* was confirmed.

In addition, based on the result of weight measurement, the tumor growth inhibition rate (IR) was calculated by the method of Equation 1 below in each experimental group.

[Equation 1]

$$IR(\%) = (1-(T1/C1)) \times 100$$

In the Equation 1, T is the average tumor weight in each experimental group, and C is the average tumor weight in the negative control group.

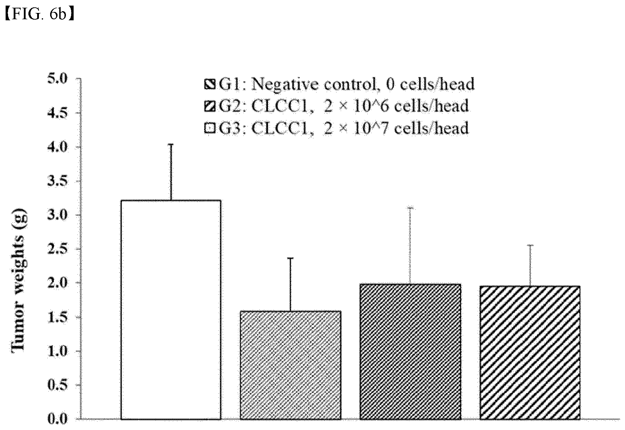

In Table 10 and FIG. 6b below, the result of measuring the tumor weight in each group after the end of the experiment of administration of the strain was shown, and in Table 11 below, the result of measuring the average body weight of mice of each group according to the progress of the experiment was shown.

[Table 10]

| Group/Dose (cells/head) | | Tumor weights (g) | IR(%) |
|---|---|---|---|
| G1 | Mean | 3.21 | 0.0 |
| 0 | S.D. | 0.83 | |
| G2 | Mean | 1.94 | 39.6 |
| 2×10$^6$ | S.D. | 0.59 | |
| | | ** | |
| G3 | Mean | 2.17 | 32 .4 |
| 2×10$^7$ | S.D. | 1.02 | |
| | | ** | |
| G4 | Mean | 1.95 | 39.3 |
| 2×10$^8$ | S.D. | 0.61 | |
| | | ** | |

\*\* p<0.01, Significant difference from the negative control group (G1) by Dunnett's t-test

[Table 11]

| Group/Dose (cells/head) | | Body weights (g) | | | | | |
| --- | --- | --- | --- | --- | --- | --- | --- |
| | | Time after administration (days) | | | | | |
| | | 1 | 8 | 15 | 22 | 29 | 36 |
| G1 | Mean | 19.5 | 19.4 | 19.4 | 19.3 | 19.8 | 19.4 |
| 0 | S .D, | 1.1 | 1.4 | 1.5 | 1.8 | 1.8 | 1.7 |
| | N | 10 | 10 | 10 | 10 | 10 | 10 |
| G2 | Mean | 20.3 | 20.0 | 19.8 | 18.9 | 19.0 | 18.8 |
| $2 \times 10^6$ | S.D. | 1.3 | 1.2 | 1.5 | 1. 1 | 1.4 | 1.3 |
| | N | 10 | 10 | 10 | 10 | 10 | 10 |
| G3 | Mean | 20.3 | 19.5 | 193 | 18.5 | 18.9 | 18.8 |
| $2 \times 10^7$ | S.D. | 0.8 | 0.9 | 1.3 | 1.5 | 1.4 | 1.3 |
| | N | 10 | 10 | 10 | 10 | 10 | 10 |
| G4 | Mean | 19.8 | 193 | 19.1 | 18.7 | 18.5 | 18.4 |
| $2 \times 10^8$ | S.D. | 1.2 | 1.4 | 1.5 | 1.4 | 1.5 | 1.6 |
| | N | 10 | 10 | 10 | 10 | 10 | 10 |

In the negative control group (G1), the tumor weight extracted after autopsy was shown as about 3.21g, but in the experimental groups, it was shown as 1.94, 2.17 and 1.95g, respectively, at the low, medium and high concentrations, and therefore the weight of the tumor tissue was shown significantly low. On the other hand, the total body weight in each group did not show a significant difference between the control group and experimental groups.

The tumor growth inhibition rate was 39.6% in the experimental group at the low concentration, and the tumor growth inhibition rate was 32.4% in the experimental group at the medium concentration, and the tumor growth inhibition rate was 39.3% in the experimental group at the high concentration, and therefore, it was confirmed that the tumor growth was inhibited 30% or more in all the experimental groups.

## (2) Necrosis and apoptosis of tumor cells

H&E staining (Hematoxylin & Eosin staining) to see necrosis of tumor cells in tumor tissue and TUNEL assay to see apoptosis were performed by conducting an autopsy after completing administration of strain CLCC1 for 36 days.

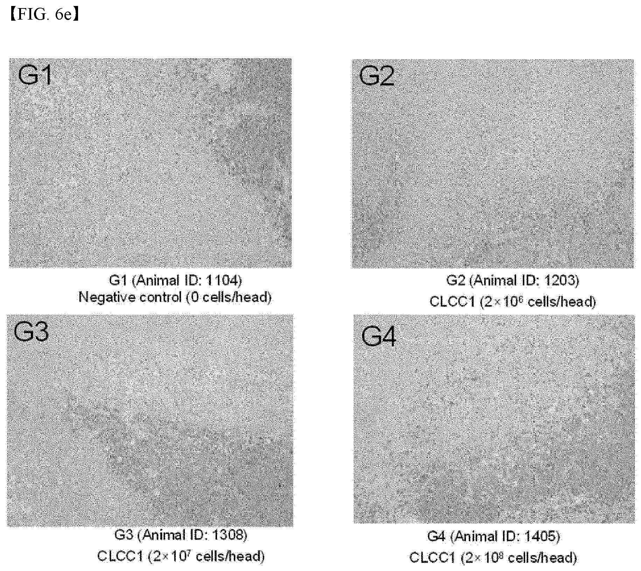

In FIG. 6e, a photograph of the H&E staining result for analysis of necrosis of tumor cells was shown. A significant difference was not observed in the level of necrosis of tumor cells between the negative control group and experimental groups. Thus, it can be found that *Faecalibacterium cancerinhibens* cell line of the present application does not induce an inflammatory response by necrosis of tumor.

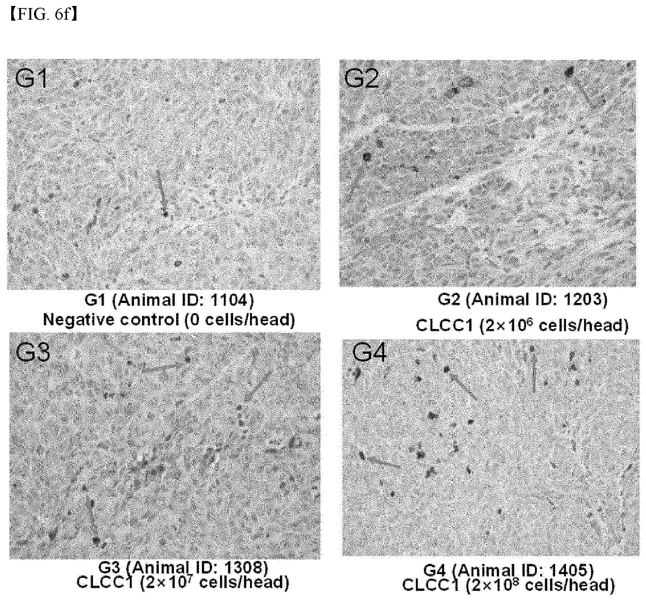

In FIG. 6f, a photograph of the TUNEL staining analysis result for analysis of apoptosis of tumor cells was shown. It was confirmed that the apoptosis of tumor cells was statistically significantly increased in the *Faecalibacterium* administered groups, compared to the negative control group. Therefore, it was confirmed that the reduction of the tumor volume in each *Faecalibacterium* administered group was not caused by tumor necrosis, but by apoptosis of tumor, and did not induce an inflammatory response.

In Table 12 below, the result of comparing the necrosis and apoptosis of tumor cells was shown. In the table below, depending on the degree of apoptosis, ± represents minimal, and + represents mild, and ++ represents moderate, and +++ represents marked, and ++++ represents severe. Below each symbol, the number of killed cells corresponding to the corresponding stage is indicated.

[Table 12]

| Group Dose (cells/head) | N | Apoptosis | | | | | | Necrosis | | | | |
| --- | --- | --- | --- | --- | --- | --- | --- | --- | --- | --- | --- | --- |
| | | Rate(%) | ± | + | ++ | +++ | ++++ | ± | + | ++ | +++ | ++++ |
| G1 / 0 | 10 | 10.33±0.68 | 5 | 5 | 0 | 0 | 0 | 0 | 0 | 0 | 10 | 0 |
| G2 / $2 \times 10^6$ | 10 | 10.33±2.56 | 4 | 6 | 0 | 0 | 0 | 0 | 0 | 3 | 7 | 0 |
| G3 / $2 \times 10^7$ | | 12.47±2.34** | 2 | 8 | 0 | 0 | 0 | 0 | 0 | 5 | 5 | 0 |

(continued)

| Group Dose (cells/head) | N | Apoptosis | | | | | | Necrosis | | | | |
|---|---|---|---|---|---|---|---|---|---|---|---|---|
| | | Rate(%) | ± | + | ++ | +++ | ++++ | ± | + | ++ | +++ | ++++ |
| G4 / $2 \times 10^8$ | 10 | 12.43±3.35 | 3 | 7 | 0 | 0 | 0 | 0 | 0 | 5 | 0 | 0 |

## p<0.01, Significant difference from the negative control group (G1) by Steel's t-test.

As can be confirmed in the Table 12, the apoptosis ratio of about 10.33% was shown in the control group (Control, G1), and the apoptosis ratio similar to the control group was shown in the experimental group at the low concentration (G2), but the apoptosis of 12.4% or more was observed in the experimental groups at the medium concentration (G3) and high concentration (G4). Accordingly, it was confirmed that the apoptosis of tumor cells was increased by administration of the *Faecalibacterium cancerinhibens* microorganism.

4-2: Evaluation of efficacy using colorectal cancer subcutaneous tumor model

To confirm the anti-cancer effect against colorectal cancer during administration of strain CLCC1, mice in which a colorectal cancer cell was subcutaneously transplanted were used, and a test was performed in Champion's oncology, a non-clinical commission testing institution in the United States. As the colorectal cancer cell, a mouse-derived colorectal cancer cell line, MC38 cell was used, and as the mouse strain, C57BL/6 was used. The MC38 cell is a cancer cell line corresponding mouse-derived colorectal carcinoma. The test group is as follows, and 10 mice were included per group.

Specifically, through a subcutaneous tumor transplantation experiment of MC38 cell line, the colorectal cancer cell line, in 6-week-old male C57BL/6 mice, colorectal cancer was caused, and the effect of the oral administration of *Faecalibacterium cancerinhibens* strain CLCC1 on the growth of colorectal cancer cells during the process of colorectal cancer occurrence was examined.

By the substantially same method as the preparation method of test formulations of the control group and experimental groups of Example 4-1, *Faecalibacterium cancerinhibens* strain CLCC1 of Example 1 was cultured in an RCM medium, and then concentrated and stored frozen to final glycerol concentration of 15%(v/v) using PBS buffer and glycerol. In addition, after hawing immediately before administration to mice, low concentration living cells at a content of 200ul per animal ($2 \times 10^7$ cells/dose) (Sample 1 corresponds to G3 of Example 8-1) and high concentration living cells ($2 \times 10^8$ cells/dose) at a content of 200ul per animal ($2 \times 10^8$ cells) (Sample 2 corresponds to G4 of Example 8-1) were orally administered. As a control group, 10 mice not administered with strain CLCC1 were used.

Sample 3 was used as high concentration dead cells ($2 \times 10^8$ cells/dose) by treating the prepared Sample 2 in an oven at a temperature of 100°C for 2 hours.

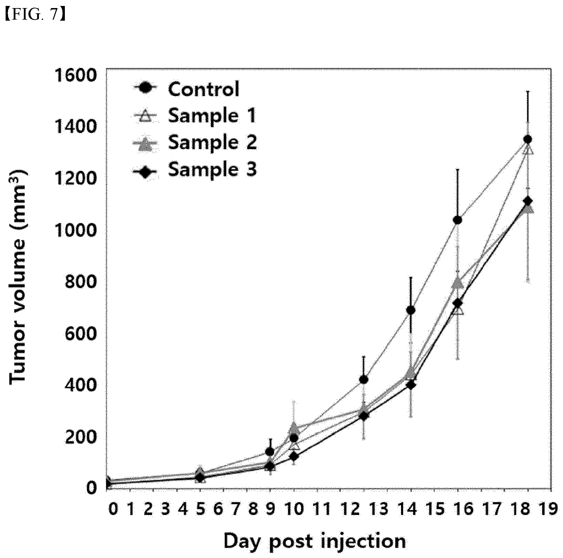

Specifically, the test formulations of the control group and Samples 1 to 3 were administered once a day for 40 days in total by starting administration 2 weeks before inoculating cancer cells. Specifically, the test formulations of the control group and Samples 1 to 3 were orally administered once a day for 40 days, and after 14 days from the start date of administration, colorectal cancer cells ($5 \times 10^5$ MC38 cells in 0.1ml PBS) were inoculated to the subcutaneous tissue of the left flank, and after a one-week engraftment period, the tumor size was observed from the 22nd day to the 40th day for 18 days. Day 22, the start date of tumor observation was regarded as Day 0, and the size of the colorectal cancer tumor (tumor volume) was measured using Magnetic Resonance (MR) imaging for 18 days. The change in the tumor size was calculated by measuring the maximum length (L) and perpendicular width (W) of tumor for 22 days after engraftment of cancer cells and introducing them to the following Equation 3. The tumor volume measured for 18 days was shown in Table 13 and FIG. 7 below.

[Equation 3]

$$Tv\ (mm^3) = L\ (mm) \times W^2\ (mm^2) \times 1/2$$

[Equation 2]

Relative reduction rate of tumor volume (%) = (1-T2/C2)) X 100

In Table 13 below, Mean means the average tumor volume and S.D. means standard deviation. Table 13 is the result of the tumor volume change in the colorectal cancer cell line-transplanted subcutaneous tumor model in which the isolated strain according to the control group and Samples 1 to 3 was administered.

[Table 13]

| Observation date | Control group | | Sample 1 | | Sample 2 | | Sample 3 | |
|---|---|---|---|---|---|---|---|---|
| | Mean | S.D. | Mean | S.D. | Mean | S.D. | Mean | S.D. |
| 1 | 28.63 | 19.64 | 18.3 | 13.12 | 29.1 | 18.21 | 19 | 14.64 |
| 4 | 47.63 | 27.39 | 44.8 | 33.41 | 60.7 | 89.62 | 42.3 | 42.41 |
| 7 | 109.38 | 60.98 | 92.6 | 59.67 | 100.6 | 111.84 | 85.5 | 89.91 |
| 8 | 174.63 | 33.93 | 171.7 | 124.72 | 231.4 | 319.62 | 124.2 | 97.38 |
| 11 | 367.63 | 83.97 | 295.2 | 201.81 | 303.8 | 360.13 | 277.2 | 265.91 |
| 13 | 633.75 | 218.75 | 437.4 | 307.75 | 446.1 | 457.75 | 396.8 | 388.22 |
| 15 | 934.13 | 329.64 | 686.6 | 495.19 | 785.5 | 694.4 | 707.4 | 679.62 |
| 18 | 1441.86 | 444.79 | 1296 | 979.09 | 1070.56 | 852.65 | 1094.89 | 897 |

As the result, the tumor volume change obtained in the groups administered with the strain of Samples 1 to 3 compared to the control group was shown in FIG. 7, respectively. Based on the last day of measurement, as the result of calculating the tumor volume of Sample 1 to Sample 3 by percentage by setting the tumor volume of the control group to 100%, Sample 1 was 89.9% (10.1% reduction), and Sample 2 was 74.2% (25.8% reduction), and Sample 3 was 75.9% (24.1 reduction), and therefore, as the test result, it was confirmed that the tumor growth was statistically significantly inhibited in the colorectal cancer model mice administered with CLCC1, compared to the control group.

It was confirmed that the tumor growth was statistically significantly inhibited even when dead cells of strain CLCC1 were administered. In addition, regarding the reduction of the tumor volume of Sample 1 and Sample 2 using living cells of strain CLCC1 strain but at different concentrations, it was confirmed that the anti-cancer activity was increased in a concentration-dependent manner under the same experimental condition.

In addition, after the day of administrating the test formulations, at an interval of 1 to 3 days, general symptoms such as appearance, body weight, behavior, excretion, and the like were observed. The test was performed for 40 days in total after administration of the test formulations, and it was observed for 18 days after engraftment of cancer cells. The measured body weight (Kg) of the experimental animals was shown in Table 14 below.

[Table 14]

| Observation date | Control group | Sample 1 | Sample 2 | Sample 3 |
|---|---|---|---|---|
| 1 | 20.60 | 20.79 | 20.60 | 21.17 |
| 4 | 20.03 | 20.62 | 21.31 | 21.64 |
| 7 | 20.24 | 20.47 | 21.42 | 21.55 |
| 8 | 20.26 | 20.41 | 21.22 | 21.52 |
| 11 | 20.05 | 20.44 | 21.06 | 21.22 |
| 13 | 20.42 | 20.44 | 21.17 | 21.28 |
| 15 | 20.54 | 20.77 | 21.13 | 21.38 |
| 18 | 20.50 | 20.93 | 21.27 | 21.09 |

For the total body weight of the experimental animals, the body weight of mice corresponding to the control group tended to be lower than that of the test groups after engraftment of cancer cells, but there was no significant difference between the control group and the test groups overall.

**Example 5: Evaluation of preventive efficacy for animal model having acute DSS-colitis**

5-1: Model animal preparation and sample administration

Female BALB/c mice (about 6 weeks old) were supplied from a supplier and an experiment was conducted after an adaptation period (about 7 days). After the adaptation period, for 7 days at the 3rd week of test substance administration, the normal control group was supplied with normal negative water, and all groups except the normal control group were provided with 3% DSS in a drinking bottle and freely supplied with water to induce colitis. Female BALB/c mice (6 weeks old) were subjected to a 6-day acclimatization period, and 6 mice (7 weeks old) per group were used in the present experiment. Test animals were conducted in a total of 6 groups, and the group composition is as follows.

[Table 15]

| Group | Group description | Dose (cells/100 uL) | Administration solution amount (mL/head) | Number of animals (subject number) |
|---|---|---|---|---|
| G1 | Normal control group | 0 | 0.2 | 6(1101~1106) |
| G2 | Negative control group | 0 | 0.2 | 6(1201~1206) |
| G3 | Control substance administration group | 200mg/Kg | 10mL/Kg | 6(1301~1306) |
| G4 | Test substance administration group 1 | $1 \times 10^7$ | 0.2 | 6(1401~1406) |
| G5 | Test substance administration group 2 | $1 \times 10^8$ | 0.2 | 6(1501~1506) |
| G7 | Test substance administration group 3 | $1 \times 10^8$ | 0.2 | 6(1701~1706) |

Control groups were set as a normal control group (G1), a negative control group (G2) and a positive control group (G3). G1 and G2 were administered with 15% glycerol as an excipient from the start of the test, and G3 was administered with 200mg/kg of Salicylazosulfapyridine (SASP). Test substance administration groups were divided into living cell groups (G4, G5) and a dead cell group (G7). The living cell groups, G4 and G5, and dead cell group (G7) were administered with CLCC1 once a day for 24 days from the start of the test to the end to confirm the preventive effect of CLCC1. Colitis was induced by putting 3% DSS in a drinking bottle and providing free water at the 3rd week of the test substance administration for 7 days.

In the present experiment, the weight loss evaluation, disease activity index (DAI) and large intestine length were measured, and histopathological examination of large intestine tissue was performed.

5-2: Weight loss evaluation

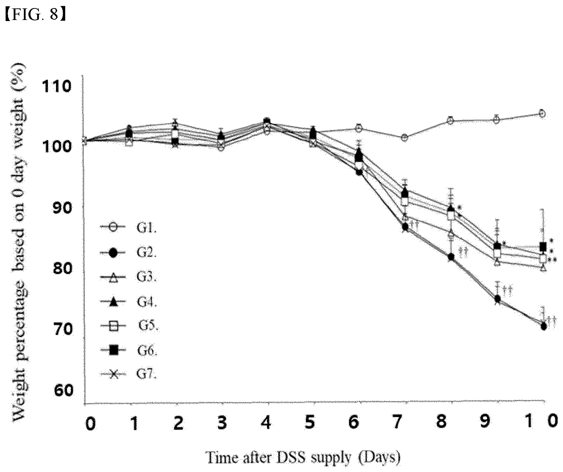

Body weight was measured twice/weekly from the start of administration to the end of the test, and it was measured before administration on each administration day. Based on the body weight measured before 3% DSS supply, the change in body weight was calculated as a percentage, and the result was shown in FIG. 8.

When DSS was supplied, the body weight began to decrease in all groups (G2-G7) after 6 days compared to before water supply, and the reduction rate tended to increase until the end of the test. Weight loss is an important and potentially dangerous side effect of inflammatory bowel disease. Compared to G2, the reduction rate of the body weight was statistically significantly reduced in G4 and G5 groups. (*p<0.05 and **p<0.01, Independent t-test) G7 showed no difference from G2. Therefore, administration of the CLCC1 living cell was confirmed to improve body weight loss due to colitis.

5-3: DAI (disease activity index)

DAI was scored according to body weight, stool consistency and presence of fecal blood from the day of 3% DSS administration and expressed as a sum. Specifically, the disease activity index was scored based on weight loss (for example, weight gain or maintenance within 1% compared to baseline (score 0); 1.5% weight loss (score 1); 5~10% weight loss (score 2); 10-15% weight loss (score 3); >15% weight loss (score 4)); stool consistency (for example, normal (score 0); loose stool (score 2); diarrhea (score 4)); and bleeding (for example, no bleeding (score 0), moderate bleeding (score 2), severe bleeding (score 4))).

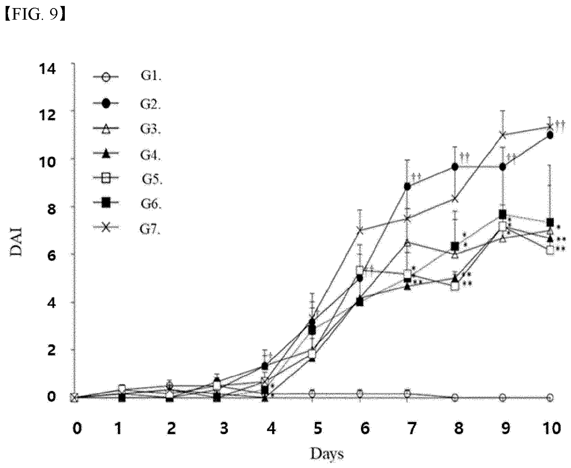

The evaluation result was shown in FIG. 9. In G3-G6, the DAI score increased after DSS administration, but at the end of the test, it showed a lower score compared to G2. At the end of the test, the average score of G3, and G4 and G6 was 7.0 points, and 6.7 points, which were statistically significantly lower than G2. (*p<0.05 and **p<0.01, Independent t-test) The DAI score of G5 was 7.3 points on average at the end of the test, which was lower than G2, but there was no statistically significant difference. The DAI score of G7 was 11.3 points on average at the end of the test, showing no difference from G2.

Therefore, it was confirmed that the DAI score was statistically significantly reduced compared to G2 due to administration of the CLCC1 living cell, and this means that symptoms such as body weight, stool condition, and presence or absence of blood in stool were improved.

### 5-4: Measurement of large intestine length

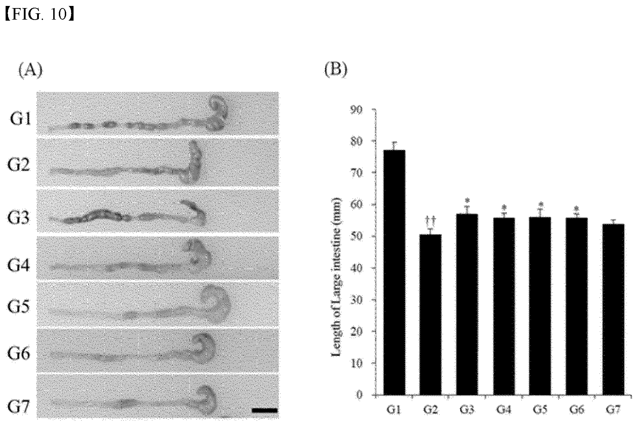

After completing the test, the animals were anesthetized, performed laparotomy, and exsanguinated, and then the large intestine tissue was extracted by individual. The extracted large intestine was photographed and its length was measured. The measured result was shown in FIG. 10.

Induced colitis with DSS reduced the length of the large intestine. The large intestine length of G2 was statistically significantly reduced compared to G1. (††p<0.01, Independent t-test) The large intestine length of the living cell administration groups G4 and G5 was reduced statistically significantly less than G2. (*p<0.05, Independent t-test) This meant that the living cell administration group inhibited large intestine atrophy compared to G2, thereby preventing or improving the decrease in length of the large intestine.

Therefore, it was confirmed that administration of the CLCC1 living cell inhibited colon atrophy and improved the decrease in length of the large intestine.

### 5-5: Analysis of cytokines

RNA was extracted from the large intestine and the gene expression of IL-6, TNF-$\alpha$ and IFN-$\gamma$ was analyzed by RT-qPCR.

The extracted large intestine tissue was washed with PBS and then stored in RNAlater (Lot No.: 00833281, Invitrogen, MA, U.S.A.). According to the manual of Total RNA extraction kit (Lot No.: 157031929, QIAGEN, Germany), total RNA was extracted. The extracted total RNA was quantified by measuring absorbance at 260 nm and 280 nm using a nucleic acid quantifier (IFNinite M200 pro, Tecan, Austria). RT-qPCR of TNF-$\alpha$, IFN-$\gamma$ and IL-6 was conducted using Real time PCR machine (CFX384, BIO-RAD, CA, U.S.A.) under the following condition.

The composition of the RT-qPCR mixture was shown in Table 16 below, and the RT-qPCR condition was shown in Table 17 below. As the RT-qPCR result, gene expression levels were compared using $2^{-\Delta\Delta Ct}$ method.

[Table 16]

| Name | Composition ($\mu$L) |
|---|---|
| iTaq universal SYBR Green reaction mix (2x) | 8.0 |
| iScript reverse transcriptase | 0.2 |
| Forward primer (3 $\mu$mole) | 1.0 |
| Reverse primer (3 $\mu$mole) | 1.0 |
| RNA (20 ng/$\mu$L) | 5.0 |
| Warer, RNase and DNase Free, PCR Certified Solution | 0.8 |
| Total | 16.0 |

[Table 17]

| Step | Temperature (°C) | Time | Cycle |
|---|---|---|---|
| Reverse Transcription | 50 | 10 min | 1 cycle |
| Pre-denaturation | 95 | 1 min | 1 cycle |

(continued)

| Step | Temperature (°C) | Time | Cycle |
|---|---|---|---|
| Denaturation | 95 | 10sec | 40 cycle |
| Annealing | 60 | 30 sec | |

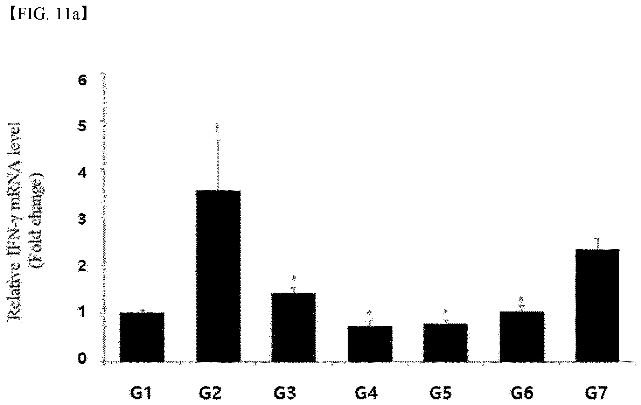

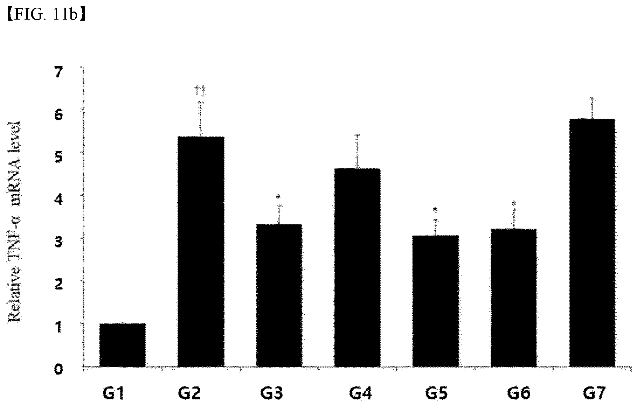

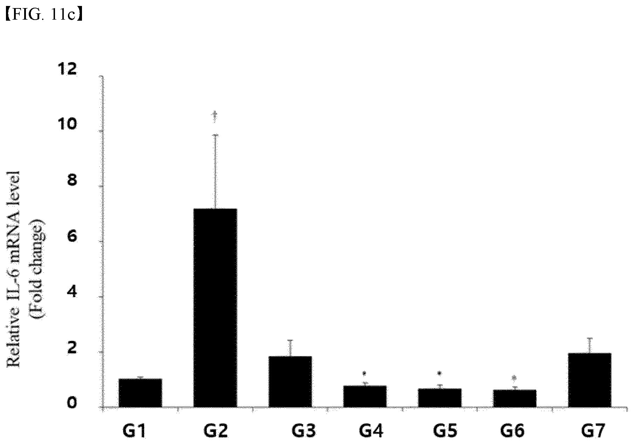

The measured result was shown in FIG. 11a to FIG. 11c.

When colitis was induced by DSS, inflammatory cytokines (TNF-$\alpha$, IL-17, IL-1$\beta$, IL-2, IFN-$\gamma$, IL-6, IL-12, IL-25, IL-33, IL-8, MCP-1, MIP-3$\alpha$, CXCL1 and IL-23, etc.) increased in expression, and anti-inflammatory cytokines (IL-4, IL-10, IL-13, IFN-$\alpha$, TGF-$\beta$, etc.) decreased in expression. Among them, in the present experiment, the expression levels of IFN-$\gamma$, IL-6, and TNF-$\alpha$ as inflammatory cytokines were measured.

In all the living cell administration groups (G4~G5), compared to the negative control group (G2), the levels of IFN-$\gamma$, and IL-6 were statistically significantly low. The TNF-$\alpha$ level was statistically significantly lower than G2 in the CLCC1 high-concentration living cell administration group (G5). (*$p < 0.05$, Independent t-test) Therefore, it was confirmed that administration of the CLCC1 living cell lowered the level of inflammatory cytokines such as IFN-y, IL-6 and TNF-$\alpha$.

5-6: Histopathological examination

The extracted tissue was fixed in 10% neutral formalin. The extracted and fixed large intestine tissue was cut into tissue sections with a thickness of 4 to 5 $\mu$m, sectioned and attached to slides. After H&E staining, it was sealed with permount (Fisher Scientific, NJ, U.S.A.), and it was observed under a microscope (CKX41, Olympus, Japan), and photographing was conducted.

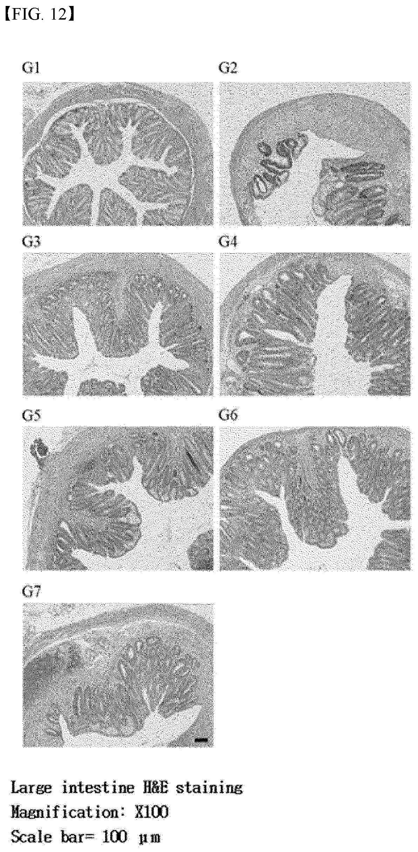

In the large intestine mucosa of the test group G2, the crypt structure was lost, and goblet cells secreting mucus were lost, and transmural inflammation occurred. A large number of red blood cells and white blood cells were observed in the tissue. In G3 ~ G5, it was observed that the crypt structure was maintained and inflammation was reduced. The observed result was shown in FIG. 12. Therefore, it was confirmed that administration of the CLCC1 living cell improved damaged intestine tissue pathology.

**Example 6: Evaluation of therapeutic efficacy for animal model having acute DSS-colitis**

6-1: Model animal preparation and sample administration

Female BALB/c mice (about 6 weeks old) were supplied from a supplier and an experiment was conducted after an adaptation period (about 7 days). After the adaptation period, for 7 days at the 3rd week of test substance administration, the normal control group was supplied with normal negative water, and all groups except the normal control group were provided with 3% DSS in a drinking bottle and freely supplied with water to induce colitis. Female BALB/c mice (6 weeks old) were subjected to a 6-day acclimatization period, and 6 mice (7 weeks old) per group were used in the present experiment. Test animals were conducted in a total of 4 groups, and the group composition is as follows.

[Table 18]

| Group | Group description | Dose (cells/100 uL) | Administration solution amount (mL/head) | Number of animals (subject number) |
|---|---|---|---|---|
| G1 | Normal control group | 0 | 0.2 | 6(1101~1106) |
| G2 | Negative control group | 0 | 0.2 | 6(1201~1206) |
| G3 | Control substance administration group | 200mg/Kg | 10mL/Kg | 6(1301~1306) |
| G6 | Test substance administration group 4 | $1 \times 10^8$ | 0.2 | 6(1601~1606) |

Control groups were set as a normal control group (G1), a negative control group (G2) and a positive control group (G3), and the treatment group (test substance administration group 4) was set as living cell group G6, respectively. G1 and G2 were administered with 15% glycerol as an excipient from the start of the test, and G3 was administered with 200mg/kg of Salicylazosulfapyridine (SASP). The test substance administration group G6 was administered with CLCC1 once a day for 10 days from the time DSS induced colitis as a living cell group to see the therapeutic effect of CLCC1. Colitis was induced by putting 3% DSS in a drinking bottle and providing free water at the 3rd week of the start of test substance administration for 7 days.

By the substantially same method as Example 5, the weight loss evaluation, disease activity index (DAI) and large intestine length were measured, and histopathological examination of large intestine tissue was performed.

6-2: Weight loss evaluation

By the substantially same method as Example 5, body weight was measured twice/weekly from the start of administration to the end of the test, and it was measured before administration on each administration day. Based on the body weight measured before 3% DSS supply, the change in body weight was calculated as a percentage, and the result was shown in FIG. 8.

When DSS was supplied, the body weight began to decrease in all groups (G2-G7) after 6 days compared to before water supply, and the reduction rate tended to increase until the end of the test. Weight loss is an important and potentially dangerous side effect of inflammatory bowel disease. Compared to G2, the reduction rate of the body weight was statistically significantly reduced in G4 and G5 groups. (*$p<0.05$ and **$p<0.01$, Independent t-test) Therefore, administration of the CLCC1 living cell was confirmed to improve body weight loss due to colitis.

6-3: DAI (disease activity index)

By the substantially same method as Example 5, DAI gave scores, and the evaluation result was shown in FIG. 9. In G6, the DAI score increased after DSS administration, but at the end of the test, it showed a lower score compared to G2. At the end of the test, the average score of G6 was 6.2 points, which was statistically significantly lower than G2. (*$p<0.05$ and **$p<0.01$, Independent t-test)

Therefore, it was confirmed that the DAI score was statistically significantly reduced compared to G2 due to administration of the CLCC1 living cell, and this means that symptoms such as body weight, stool condition, and presence or absence of blood in stool were improved.

6-4: Measurement of large intestine length

By the substantially same method as Example 5, the large intestine tissue of the experimental animals was extracted, and the extracted large intestine was photographed and the length was measured. The measured result was shown in FIG. 10. Induced colitis with DSS reduced the length of the large intestine.

The large intestine length of G2 was statistically significantly reduced compared to G1. (††$p<0.01$, Independent t-test) The large intestine length of the living cell administration group G6 was reduced statistically significantly less than G2. (*$p<0.05$, Independent t-test) This meant that the living cell administration group inhibited large intestine atrophy compared to G2, thereby preventing or improving the decrease in length of the large intestine.

Therefore, it was confirmed that administration of the CLCC1 living cell inhibited colon atrophy and improved the decrease in length of the large intestine.

6-5: Cytokines Analysis

By the substantially same method as Example 5, RNA was extracted from the large intestine and the gene expression of IL-6, TNF-$\alpha$ and IFN-$\gamma$ was analyzed by RT-qPCR. The measured result was shown in FIG. 11a to FIG. 11c.

When colitis was induced by DSS, inflammatory cytokines (TNF-$\alpha$, IL-17, IL-1$\beta$, IL-2, IFN-$\gamma$, IL-6, IL-12, IL-25, IL-33, IL-8, MCP-1, MIP-3$\alpha$, CXCL1 and IL-23, etc.) increased in expression, and anti-inflammatory cytokines (IL-4, IL-10, IL-13, IFN-$\alpha$, TGF-$\beta$, etc.) decreased in expression. Among them, in the present experiment, the expression levels of IFN-$\gamma$, IL-6, and TNF-$\alpha$ as inflammatory cytokines were measured.

In all the living cell administration group (G6), compared to the negative control group (G2), the levels of IFN-$\gamma$, and IL-6 were statistically significantly low. The TNF-$\alpha$ level was statistically significantly lower than G2 in the CLCC1 high-concentration living cell administration group (G6). (*$p<0.05$, Independent t-test) Therefore, it was confirmed that administration of the CLCC1 living cell lowered the level of inflammatory cytokines such as IFN-y, IL-6 and TNF-$\alpha$.

6-6: Histopathological examination

By the substantially same method as Example 5, the sections of the extracted and fixed large intestine tissue were observed with a (CKX41, Olympus, Japan), and photographing was conducted.

In the large intestine mucosa of the test group G2, the crypt structure was lost, and goblet cells secreting mucus were lost, and transmural inflammation occurred. A large number of red blood cells and white blood cells were observed in the tissue. In G6, it was observed that the crypt structure was maintained and inflammation was reduced. The observed result was shown in FIG. 12. Therefore, it was confirmed that administration of the CLCC1 living cell improved damaged intestine tissue pathology.

**Example 7: Evaluation of reduction of inflammatory cytokines (HT-29 and RAW 264.7)**

7-1: Culture of HT-29 and RAWN 264.7 cell lines

HT-29 (Korean Cell Line Bank, KCLB 30038) and RAW 264.7 (Korean Cell Line Bank, KCLB 40071) cells were cultured in RPMI 1640 medium in which 10% FBS (Fetal bovine serum), penicillin (100 $\mu$g/mL), and streptomycin(100 $\mu$g/mL) were added under the 37°C, 5% $CO_2$ conditions, and subculturing was carried out once per 2~3 days.

7-2: Culturing and recovering of strain

*F.prausnitzii* A2-165 used in the present experiment was obtained from DSMZ (DSMZ collection, Braunschweig, Germany) (DSM N°17677).

The used *Faecalibacterium* spp. microorganisms, *Fcancerinhibens* CLCC1 and *F.prausnitzii* A2-165 are obligate anaerobic strains, and all the culturing processes were carried out in an anaerobic chamber, and cultured in media of mRCM, and activated by subculture at 18 to 23 hour intervals twice in total, and the used in the experiment. The obtained culturing solution was centrifuged under the condition of 3200 $\times$ g, 5 min, and the supernatant except for precipitate was recovered and used in the experiments of Example 7 and Example 8.

7-3: Efficacy evaluation for IL-8 in HT-29 cells

Evaluation of Immuno-Modulatory Properties Using HT-29 Cells was performed (ELISA).

Specifically, using human large intestine epithelial cells, HT-29, after culturing them in a 24-well plate by $5\times10^4$ Cells /well, one day before co-culture with the supernatant of CLCC1 and A2-165, it was changed to a medium which 10% FBS was comprised in RPMI1640. On the day of co-culture, to the RPMI1640 (+10% FBS) medium, CLCC1 and A2-165 supernatant or media of mRCM was added to be 10%. After treating TNF-$\alpha$ (5~10ng/ml; Peprotech, NJ) in a 37°C, 5% $CO_2$ incubator for 6 hours, culture was performed. All samples were performed in triplicate. After the co-culture, the culture supernatant was collected, and Interleukin-8 (IL-8) was measured by ELISA (Invitrogen, USA). The result was expressed in pg/m$\ell$, and normalization was performed using the IL-8 reference value using only one treated only media of mRCM as a negative control.

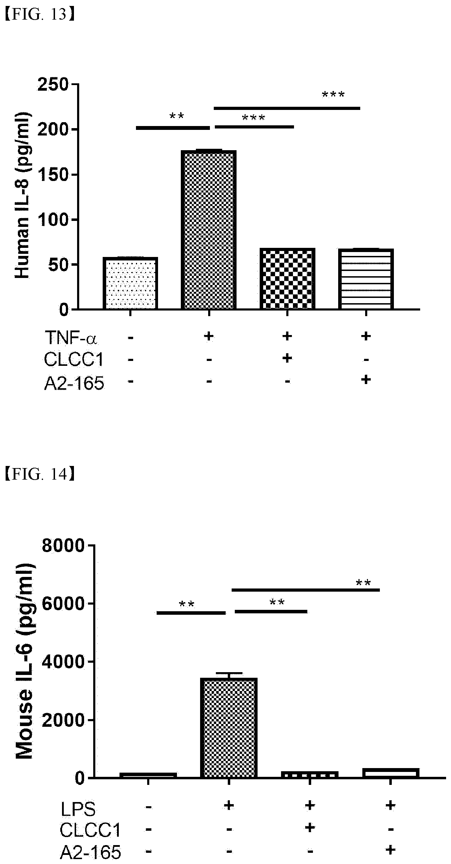

The secretion amount of human IL-8 from the HT-29 cells analyzed by ELISA was shown in FIG. 13. Through this examination, it could be confirmed that IL-8 was statistically significantly reduced, in the supernatants of *Fcancerinhibens* CLCC1 and *F.prausnitzii* A2-165, compared to the control group treated only with media of mRCM from the HT-29 cells.

This indicates that *Fcancerinhibens* CLCC1 can reduce an inflammatory response increased by TNF-$\alpha$, and in addition, it means that it can be utilized as a therapeutic agent of inflammatory bowel disease, by inhibiting infiltration of the intestinal mucosa by Neutrophil and secretion of inflammatory mediators (FIG. 13).

7-4: Efficacy evaluation for IL-6 and TNF-$\alpha$ in RAW 264.7 cells

Evaluation of Immuno-Modulatory Properties Using RAW 264.7 was performed. (ELISA)

Specifically, using a mouse macrophage-derived cell line, RAW 264.7, after culturing them in a 24-well plate by $5\times10^4$ Cells /well, one day before co-culture with the supernatant of *Fcancerinhibens* CLCC1 and A2-165, it was changed to a medium which 10% FBS was comprised in RPMI1640. On the day of co-culture, to the RPMI1640 (+10% FBS) medium, CLCC1 and A2-165 supernatant or media of mRCM was added to be 10%. After treating LPS (100ng/ml; Sigma, L4391) in a 37°C, 5% $CO_2$ incubator for 6 hours, culture was performed. All samples were performed in triplicate. After the co-culture, the culture supernatant was collected, and Interleukin-6 (IL-6) and TNF-$\alpha$ were measured by ELISA (Invitrogen, USA). The result was expressed in pg/mi, and normalization was performed using the IL-6 and TNF-$\alpha$ reference values using only one treated only media of mRCM as a negative control.

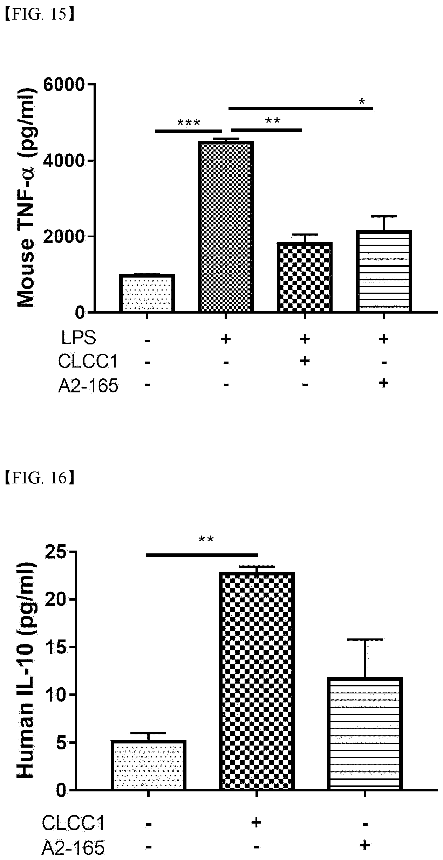

The secretion amount of mouse IL-6 and TNF-$\alpha$ from the RAW 264.7 cells analyzed by ELISA was shown in FIG. 14 and FIG. 15. Through this examination, it could be confirmed that IL-6 and TNF-$\alpha$ were statistically significantly reduced, in the supernatants of *Fcancerinhibens* CLCC1 and *F.prausnitzii* A2-165, compared to the control group treated only with media of mRCM from the RAW 264.7 cells.

This indicates that CLCC1 can reduce an inflammatory response increased by LPS, and improvement of inflammation can be expected by inhibiting secretion of RORyT or IL-17 according to Th17 cell differentiation which can be induced by IL-6, and in addition, it meant that it can be utilized as a therapeutic agent of inflammatory bowel disease, by involving in expression of inflammation-related cytokines through regulation of various immunocytes in TNF-$\alpha$ (FIGs. 14 and 15).

**Example 8: Evaluation of inhibitory activity of inflammatory cytokines (HPBMC)**

8-1: HPBMC Culture

HPBMC (Lonza, 4W-270) was cultured in LGM-3™ (Lonza, CC-3211) medium in which 10% FBS (Fetal bovine serum) was added under the 37°C, 5% $CO_2$ condition, thereby conducting an experiment.

8-2: Efficacy evaluation for human IL-10 in HPBMC

Evaluation of Immuno-Modulatory Properties Using HPBMC was performed.

Specifically, after culturing Human Peripheral Blood Mononuclear Cells (HPBMC) in a 12-well plate by $1 \times 10^6$ Cells /well, the supernatants of CLCC1 and A2-165 and PBS were added to be 10%. After the reaction in a 37°C, 5% CO2 incubator for 24 hours, culture was performed. All samples were performed in triplicate. After the co-culture, the culture supernatant was collected, and Interleukin-10 (IL-10) was measured by ELISA (Invitrogen, USA). The result was expressed in pg/mi, and normalization was performed using the IL-10 reference value using only one treated only PBS as a negative control.

The result of the analyzed efficacy for human IL-10 in HPBMC was shown in FIG. 16. Through this examination, it could be confirmed that IL-10 was statistically significantly increased in the CLCC1 supernatant, by comparing the supernatants of *Fcancerinhibens* CLCC1 and *F.prausnitzii* A2-165 to the control group treated only with PBS in HPBMC.

This indicates that *Fcancerinhibens* CLCC1 can exert an anti-inflammatory action by involving in expression of IL-10 among anti-inflammation-related cytokines in human peripheral blood mononuclear cells, and this means that it can be utilized as a therapeutic agent of inflammatory bowel disease (FIG. 16).

8-3: Efficacy evaluation for IL-6 in HPBMC

Evaluation of Immuno-Modulatory Properties Using HPBMC was performed.

Specifically, after culturing Human Peripheral Blood Mononuclear Cells (HPBMC) in a 24-well plate by $1 \times 10^6$ Cells /well, the CLCC1 supernatant and PBS were added to be 10%. After treating LPS (100ng/ml; Sigma, L4391) in a 37°C, 5% CO2 incubator for 6 hours, culture was performed. All samples were performed in triplicate. After the co-culture, the culture supernatant was collected, and Interleukin-6 (IL-6) was measured by ELISA (Invitrogen, USA). The result was expressed in pg/m$\ell$, and normalization was performed using the IL-6 reference value using only one treated only PBS as a negative control.

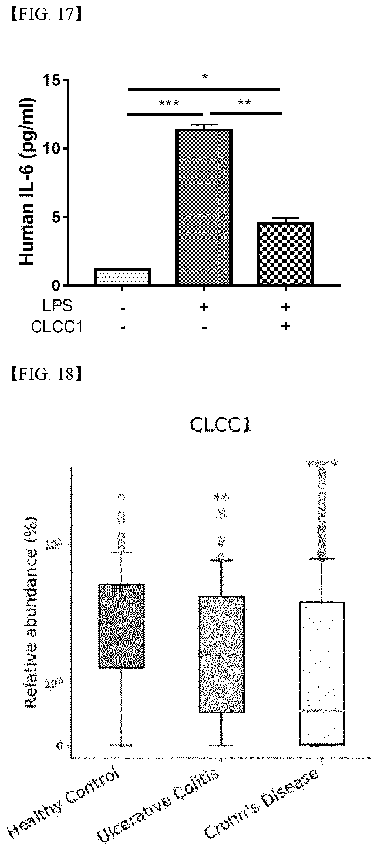

The result of the analyzed efficacy for human IL-6 in HPBMC was shown in FIG. 17. Through this examination, it could be confirmed that IL-6 was statistically significantly reduced in the *F.cancerinhibens* CLCC1 supernatant, compared to the control group treated only with PBS in HPBMC.

This indicates that *F.cancerinhibens* CLCC1 can inhibit an inflammatory response by involving in expression of IL-6 among inflammation-related cytokines in human peripheral blood mononuclear cells, and this means that it can be utilized as a therapeutic agent of inflammatory bowel disease (FIG. 17).

**Example 9: Microorganism detection using PCR**

9-1: Preparation of samples and primers

Please briefly describe sample preparation. The strain genomic DNA was prepared using DNA prep kit (e.g., MO BIO's PowerSoil DNA Isolation Kit).

Through analysis using blastn program, it was confirmed that Site-specific DNA-methyltransferase (dam) was specific to *F.cancerinhibens* CLCCL as it was not found in other microorganisms. In order to confirm *Fcancerinhibens* CLCCL-specific detection more easily and quickly and accurately in real time, based on the dam gene sequence, real-time PCR amplification reaction was performed on the sample using a primer pair consisting of forward and reverse primers.

9-2: Detection using curve value (Ct value)

Real-time PCR amplification reaction was performed using Bio-rad's CFX96 Real-time PCR system (Bio-Rad laboratories, Inc., USA), and the real-time PCR amplification reaction was conducted in a total amount of $20\mu\ell$ with a real-time PCR mixed solution containing SYBR premix Ex Taq (2x) (TAKARA Bio, Inc., Japan) and 10 pM of each of the above primers. The amount of genomic DNA of the strains added to the real-time PCR mixed solution was about 25 ng. The real-time PCR amplification reaction was denaturing at 95°C for 30 seconds, repeating 40 cycles at 95°C for 5 seconds, and at 60°C for 30 seconds, and then reacting at 95°C for 15 seconds. Thereafter, it was increased by 0.5°C per 5 seconds to 65~95°C to derive a melting curve and a melting peak. Relative Fluorescence Units (RFU) were measured according to the number of cycles.

The curve value (Ct value) was specifically confirmed only in the sample comprising *F.cancerinhibens* CLCCL DNA, and from the above result, it could be seen that the primer set and probe of the present invention could specifically detect *F.cancerinhibens*.

9-3: Quantitative analysis

Using a Site-specific DNA-methyltransferase (dam) gene, using a substance (e.g., plasmid, or genomic DNA) for which the amount of *Faecalibacterium cancerinhibens* was already measured, a standard curve was set, and then it was utilized to measure the concentration.

For the amount of total bacteria, after extracting the genome of all microorganisms in the fecal sample using a 16S universal primer, like this, using a substance of which amount was already measured (e.g., plasmid, genomic DNA), a standard curve was set, and then it was utilized to measure the concentration.

For relative quantification, a value of dividing the *Fcancerinhibens* CLCCL amount calculated above by the amount of total bacteria was used.

**Example 10: Microorganism detection using metagenome analysis**

The data used in the analysis is data obtained by performing the shotgun metagenome sequencing method on fecal microorganisms, and among the data used in the analysis, in the case of Colorectal Cancer and the healthy group compared to Colorectal Cancer group (data of which SampleId starts with ERR among healthy controls), open data published as a parper (https://gut.bmj.com/content/66/1/70) were downloaded, and in case of other ulcerative colitis, Crohn's disease and healthy group data, fecal microorganism shotgun metagenome sequence data collected independently through joint research were used.

Each sample data was analyzed by K-mer exact matching method (Korean Patent Publication No. 10-2020-0027900) for a core gene which calculates abundance of microbial species at the species level, thereby obtaining a profile of the abundance of microbial species in the sample. For analysis of the abundance of the *Fcancerinhibens* CLCCL strain, first, in order to confirm that it is distinguished from the existing genome at the species level, ANI (Average Nucleotide Frequency) analysis was performed with the genome of *Faecalibacterium prausnitzii* ATCC 27768, it was confirmed that it was a novel species with a significantly low value of 85.74 compared to the standard similarity of 95 at the species level, and therefore, it was added to the reference microbial genome database.

For each sample, the distribution in each disease group and healthy group was compared using the reference database comprising the genome of *Faecalibacterium cancerinhibens* CLCC1. The experimental result was shown in Table 19 and FIG. 18 below.

[Table 19]

| Classification | Mean | Standard deviation | Minimal value | Maximal value | p-value |
|---|---|---|---|---|---|
| Healthy control group | 3.589 | 4.315 | 0 | 26.521 | - |
| Ulcerative Colitis | 2.363 | 2.980 | 0 | 19.872 | 0.006402 |
| Crohn's Disease | 3.436 | 7.651 | 0 | 55.056 | 6.71E-06 |

The CLCC1 strain according to the present invention showed significantly different results between the healthy group and ulcerative colitis group, and the healthy group and Crohn's disease, and this shows that disease groups can be classified by the difference in the amount of distribution while residing as an intestinal microorganism.

[ACCESSION NUMBER]

Name of depository authority : Korean Culture Center of Microorganisms (overseas)
Accession number : KCCM13783BP
Accession date : 20190103

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

## RECEIPT IN THE CASE OF AN ORIGINAL DEPOSIT

issued pursuant to Rule 7.1

TO: CHUN, Jongsik

ChunLab, Inc.,
2477, Nambusunhwan-ro, Seocho-gu, Seoul
Republic of Korea

| I.  IDENTIFICATION OF THE MICROORGANISM | |
|---|---|
| Identification reference given by the DEPOSITOR:<br><br>*Faecalibacterium* sp. CLCC1 | Accession number given by the INTERNATIONAL DEPOSITARY AUTHORITY:<br><br>**KCTC 13783BP** |

| II.  SCIENTIFIC DESCRIPTION AND/OR PROPOSED TAXONOMIC DESIGNATION |
|---|
| The microorganism identified under I above was accompanied by:<br><br>[  ] a scientific description<br><br>[  ] a proposed taxonomic designation<br><br>(Mark with a cross where applicable) |

| III.  RECEIPT AND ACCEPTANCE |
|---|
| This International Depositary Authority accepts the microorganism identified under I above, which was received by it on **January 3, 2019.** |

| IV.  RECEIPT OF REQUEST FOR CONVERSION |
|---|
| The microorganism identified under I above was received by this International Depositary Authority on and a request to convert the original deposit to a deposit under the Budapest Treaty was received by it on |

| V.  INTERNATIONAL DEPOSITARY AUTHORITY | |
|---|---|
| Name: **Korean Collection for Type Cultures**<br><br>Address: Korea Research Institute of Bioscience and Biotechnology (KRIBB) 181, Ipsin-gil, Jeongeup-si, Jeollabuk-do 56212 Republic of Korea | Signature(s) of person(s) having the power to represent the International Depositary Authority or of authorized official(s):<br><br>*Song-Gun Kim*<br><br>KIM, Song-Gun, Director Date: **January 3, 2019** |

Form BP/4 (KCTC Form 37)                                                                                                                          sole page

**Claims**

1.  A composition for prevention or treatment of inflammatory disease, comprising at least one selected from the group consisting of; a microbial cell of a bacterium comprising a 16S rRNA sequence having 98% or more sequence identity with a nucleotide sequence of SEQ ID NO: 1; a culture of the microorganism; a lysate of the microorganism; and at least an extract selected from the group consisting of the microbial cell, culture and lysate.

2. The composition according to claim 1, wherein the inflammatory disease is inflammatory gastrointestinal disease.

3. The composition according to claim 2, wherein the inflammatory gastrointestinal disease is ulcerative colitis (UC) or Crohn's disease (CD).

4. The composition according to claim 1, wherein the bacterium has an immunomodulatory activity or an anti-inflammatory activity.

5. The composition according to claim 1, wherein the bacterium has an immunomodulatory activity by modulating cytokine.

6. The composition according to claim 5, wherein the cytokine is at least one selected from the group consisting of TNF-$\alpha$, IL-6, IL-8 and IL-10.

7. The composition according to claim 1, wherein the bacterium is comprised in $1\times10^3$ to $1\times10^{15}$ CFU/g based on the total weight of the composition.

8. The composition according to claim 1, wherein the bacterium is live or lyophilizate.

9. The composition according to claim 1, wherein the composition further comprises at least one drug selected from the group consisting of anti-inflammatory agents, corticosteroids, immunomodulators, antibiotics, tumor necrosis factor inhibitors, Janus kinase inhibitors, anti-integrin agents, and interleukin inhibitors.

10. The composition according to claim 1, wherein the bacterium has at least one selected from the following characteristics:

   not including 17:0 iso 3OH fatty acid,
   a tumor growth inhibitory activity or anti-cancer activity causing apoptosis of cancer cells,
   a relative reduction rate of tumor volume of 5% or more compared to the control group not being administered by the microorganism, when the microorganism is administered into an animal model transplanted with a cancer cell line,
   a tumor growth inhibition rate of tumor weight of 5% or more compared to a control group not being administered by the microorganism, when the microorganism is administered into an animal model transplanted with a cancer cell line, and
   being cultured under temperature of 25 to 40°C and anaerobic conditions.

11. The composition according to claim 1, wherein the bacterium is *Faecalibacterium cancerinhibens* deposited with Accession number KCTC 13783BP.

12. The composition according to claim 1, wherein the composition further comprises a pharmaceutically acceptable excipient, carrier or diluent.

13. The composition according to claim 1, wherein the composition is a capsule, tablet, granule, powder, troche, suppository or suspension.

14. The composition according to claim 1, wherein the composition is probiotics.

15. The composition according to claim 1, wherein the composition comprises probiotics and prebiotics.

16. A method for prevention or treatment of inflammatory bowel disease (IBD), comprising administering at least one selected from the group consisting of; a microbial cell of a bacterium comprising a 16S rRNA sequence having 98% or more sequence identity with a nucleotide sequence of SEQ ID NO: 1; a culture of the microorganism; a lysate of the microorganism; and at least an extract selected from the group consisting of the microorganism, culture and lysate into a subj ect.

【FIG. 1a】

【FIG. 1b】

【FIG. 2a】

【FIG. 2b】

【FIG. 2c】

| | OrthoANIu (%) |
| Faecalibacterium sp. CLCC1 | |
| Faecalibacterium prausnitzii ATCC 27768[T] | |
| Subdoligranulum variabile DSM 15176[T] | |
| Gemmiger formicilis ATCC 27749[T] | |
| Ruthenibacterium lactatiformans 585-1[T] | |
| Intestinimonas butyriciproducens SRB-521-5-I[T] | |
| Intestinimonas massiliensis GD2[T] | |
| Fournierella massiliensis AT2[T] | |
| Pseudoflavonifractor capillosus ATCC 29799[T] | |

84    88    92    96

【FIG. 2d】

**Comparison Table of ANU values of strains** (Comparison of average nucleotide identity)

| | 1 | 2 | 3 | 4 | 5 | 6 | 7 | 8 | 9 |
|---|---|---|---|---|---|---|---|---|---|
| 1 | 100.00 | 85.99 | 71.86 | 67.41 | 68.78 | 69.34 | 70.49 | 72.73 | 74.50 |
| 2 | 85.99 | 100.00 | 72.49 | 69.06 | 69.22 | 70.58 | 71.34 | 72.94 | 75.81 |
| 3 | 71.86 | 72.49 | 100.00 | 69.43 | 73.69 | 76.62 | 74.94 | 73.44 | 72.01 |
| 4 | 67.41 | 69.06 | 69.43 | 100.00 | 77.58 | 74.62 | 67.10 | 68.92 | 67.74 |
| 5 | 68.78 | 69.22 | 73.69 | 77.58 | 100.00 | 77.52 | 71.59 | 72.95 | 68.57 |
| 6 | 69.34 | 70.58 | 76.62 | 74.62 | 77.52 | 100.00 | 72.66 | 74.07 | 69.39 |
| 7 | 70.49 | 71.34 | 74.94 | 67.10 | 71.59 | 72.66 | 100.00 | 71.81 | 70.68 |
| 8 | 72.73 | 72.94 | 73.44 | 68.92 | 72.95 | 74.07 | 71.81 | 100.00 | 78.17 |
| 9 | 74.50 | 75.81 | 72.01 | 67.74 | 68.57 | 69.39 | 70.68 | 78.17 | 100.00 |

1, CLCC1; 2, Faecalibacterium prausnitzii ATCC 27768(T); 3, Fournierella massiliensis AT2(T); 4, Intestinimonas butyriciproducens DSM 26588(T); 5, Intestinimonas massiliensis GD2(T); 6, Pseudoflavonifractor capillosus ATCC 29799(T); 7, Ruthenibacterium lactatiformans 585-1(T); 8, Subdoligranulum variabile DSM 15176(T); 9, Gemmiger formicilis ATCC 27749(T)

【FIG. 3a】

(A) *Faecalibacterium* sp. CLCC1

[FIG. 3b]

EP 4 219 681 A1

**(B)** *Faecalibacterium prausnitzii* ATCC 27768[T]

【FIG. 3c】

## (A) *Faecalibacterium* sp. CLCC1

【FIG. 3d】

## (B) *Faecalibacterium prausnitzii* ATCC 27768[T]

【FIG. 3e】

【FIG. 3f】

【FIG. 4a】

【FIG. 4b】

【FIG. 4c】

【FIG. 5a】

| Days after administration | Control | | | | CLCC1 | | | |
|---|---|---|---|---|---|---|---|---|
| | 1 | 2 | 3 | 4 | 1 | 2 | 3 | 4 |
| 0 | 11.1 | 12.1 | 15.9 | 9.5 | 9.4 | 21.1 | 13.7 | 12.9 |
| 4 | 21.7 | 29.5 | 41.4 | 26.8 | 20.2 | 46.1 | 19.5 | 27.7 |
| 11 | 63.8 | 37.9 | 58.4 | 61.1 | 36.5 | 88.2 | 34.7 | 45.7 |
| 18 | 105.2 | 12.2 | 33.0 | 64.1 | 14.6 | 62.5 | 14.2 | 23.1 |
| 25 | 124.8 | 6.1 | 33.8 | 101.9 | 8.6 | 61.0 | 7.1 | 8.4 |
| 32 | 162.3 | 0.0 | 34.3 | 164.1 | 11.0 | 126.0 | 0.0 | 0.0 |
| 39 | 174.8 | 0.0 | 48.5 | 243.0 | 18.7 | 154.2 | 0.0 | 0.0 |

【FIG. 5b】

【FIG. 5c】

| Days after administration | Control | | | | CLCC1 | | | |
|---|---|---|---|---|---|---|---|---|
| | 1 | 2 | 3 | 4 | 1 | 2 | 3 | 4 |
| 0 | 0 | 0.0 | 0.0 | 0.0 | 0.0 | 0.0 | 0.0 | 0.0 |
| 4 | 1.0 | 1.4 | 1.6 | 1.8 | 1.2 | 1.2 | 0.4 | 1.2 |
| 11 | 1.9 | 0.3 | 0.4 | 1.3 | 0.8 | 0.9 | 0.8 | 0.7 |
| 18 | 0.6 | -0.7 | -0.4 | 0.0 | -0.6 | -0.3 | -0.6 | -0.5 |
| 25 | 0.2 | -0.5 | 0.0 | 0.6 | -0.4 | -0.0 | -0.5 | -0.6 |
| 32 | 0.3 | -1.0 | 0.0 | 0.6 | 0.3 | 1.1 | -1.0 | -1.0 |
| 39 | 0.1 | 0.0 | 0.4 | 0.5 | 0.7 | 0.2 | 0.0 | 0.0 |

【FIG. 6a】

【FIG. 6b】

【FIG. 6c】

【FIG. 6d】

【FIG. 6e】

G1 (Animal ID: 1104)
Negative control (0 cells/head)

G2 (Animal ID: 1203)
CLCC1 ($2 \times 10^6$ cells/head)

G3 (Animal ID: 1308)
CLCC1 ($2 \times 10^7$ cells/head)

G4 (Animal ID: 1405)
CLCC1 ($2 \times 10^8$ cells/head)

【FIG. 6f】

G1 (Animal ID: 1104)
Negative control (0 cells/head)

G2 (Animal ID: 1203)
CLCC1 ($2 \times 10^6$ cells/head)

G3 (Animal ID: 1308)
CLCC1 ($2 \times 10^7$ cells/head)

G4 (Animal ID: 1405)
CLCC1 ($2 \times 10^8$ cells/head)

【FIG. 7】

【FIG. 8】

【FIG. 9】

【FIG. 10】

(A)

(B)

【FIG. 11a】

【FIG. 11b】

【FIG. 11c】

【FIG. 12】

Large intestine H&E staining
Magnification: X100
Scale bar= 100 μm

【FIG. 13】

【FIG. 14】

【FIG. 15】

| LPS | - | + | + | + |
| CLCC1 | - | - | + | - |
| A2-165 | - | - | - | + |

【FIG. 16】

| CLCC1 | - | + | - |
| A2-165 | - | - | + |

【FIG. 17】

【FIG. 18】

| | INTERNATIONAL SEARCH REPORT | International application No. |
|---|---|---|
| | | **PCT/KR2021/013152** |

**A.    CLASSIFICATION OF SUBJECT MATTER**

**C12N 1/20**(2006.01)i; **C12Q 1/6883**(2018.01)i; **C12Q 1/689**(2018.01)i; **G01N 33/68**(2006.01)i; **G01N 33/569**(2006.01)i; **A61K 35/74**(2006.01)i; **A61P 1/00**(2006.01)i; C12R 1/01(2006.01)n

According to International Patent Classification (IPC) or to both national classification and IPC

**B.    FIELDS SEARCHED**

Minimum documentation searched (classification system followed by classification symbols)

C12N 1/20(2006.01); A61K 35/74(2006.01); A61K 35/741(2014.01); A61K 47/36(2006.01); A61P 1/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 패칼리박테리움(Faecalibacterium), 염증성 위장관 질환(IBD, inflammatory bowel disease), 궤양성 대장염(ulcerative colitis), 크론병(Crohn's disease)

**C.    DOCUMENTS CONSIDERED TO BE RELEVANT**

| Category* | Citation of document, with indication, where appropriate, of the relevant passages | Relevant to claim No. |
|---|---|---|
| Y | NCBI. GenBank accession no. MF185398.1. Faecalibacterium prausnitzii strain CNCM_I_4540 16S ribosomal RNA gene, partial sequence. 08 June 2017.<br>    See the sequences. | 1-15 |
| Y | KR 10-2015-0134356 A (RIJKSUNIVERSITEIT GRONINGEN et al.) 01 December 2015 (2015-12-01)<br>    See paragraphs [0001], [0002], [0006], [0020], [0021] and [0037]; and claims 1, 2, 4, 7, 9 and 13. | 1-15 |
| Y | US 2019-0030089 A1 (INSTITUT NATIONAL DE LA RESEARCHE AGRONOMIQUE (INRA) et al.) 31 January 2019 (2019-01-31)<br>    See paragraphs [0001], [0007]-[0016], [0043]-[0050], [0058] and [0070]; and claims 1, 4-6, 11, 14 and 15. | 1-15 |
| A | FERREIRA-HALDER, C. V. et al. Action and function of Faecalibacterium prausnitzii in health and disease. Best Practice & Research Clinical Gastroenterology. 2017, vol. 31, pp. 643-648.<br>    See entire document. | 1-15 |

☑ Further documents are listed in the continuation of Box C.      ☑ See patent family annex.

| * Special categories of cited documents: | "T" later document published after the international filing date or priority date and not in conflict with the application but cited to understand the principle or theory underlying the invention |
|---|---|
| "A"  document defining the general state of the art which is not considered to be of particular relevance | |
| "D"  document cited by the applicant in the international application | "X" document of particular relevance; the claimed invention cannot be considered novel or cannot be considered to involve an inventive step when the document is taken alone |
| "E"  earlier application or patent but published on or after the international filing date | |
| "L"  document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O"  document referring to an oral disclosure, use, exhibition or other means | "&" document member of the same patent family |
| "P"  document published prior to the international filing date but later than the priority date claimed | |

| Date of the actual completion of the international search | Date of mailing of the international search report |
|---|---|
| **23 December 2021** | **23 December 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/013152** |

**C.    DOCUMENTS CONSIDERED TO BE RELEVANT**

| Category* | Citation of document, with indication, where appropriate, of the relevant passages | Relevant to claim No. |
| --- | --- | --- |
| A | US 2017-0348360 A1 (CRESTOVO LLC) 07 December 2017 (2017-12-07)<br>    See entire document. | 1-15 |

Form PCT/ISA/210 (second sheet) (July 2019)

**INTERNATIONAL SEARCH REPORT**

| International application No. |
| --- |
| **PCT/KR2021/013152** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**

| International application No. |
| --- |
| **PCT/KR2021/013152** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **16**
   because they relate to subject matter not required to be searched by this Authority, namely:

   Claim 16 pertains to a method for treatment of the human body, and thus pertains to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2019)

**INTERNATIONAL SEARCH REPORT**
Information on patent family members

International application No.

**PCT/KR2021/013152**

| Patent document cited in search report | | | Publication date (day/month/year) | Patent family member(s) | | | Publication date (day/month/year) |
|---|---|---|---|---|---|---|---|
| KR | 10-2015-0134356 | A | 01 December 2015 | AU | 2014-226633 | A1 | 03 September 2015 |
| | | | | BR | 112015020819 | A2 | 18 July 2017 |
| | | | | CN | 105228635 | A | 06 January 2016 |
| | | | | EP | 2988761 | A1 | 02 March 2016 |
| | | | | HK | 1214954 | A1 | 12 August 2016 |
| | | | | JP | 2016-511272 | A | 14 April 2016 |
| | | | | MX | 2015011700 | A | 20 July 2016 |
| | | | | RU | 2015137415 | A | 10 April 2017 |
| | | | | US | 2016-0000838 | A1 | 07 January 2016 |
| | | | | WO | 2014-137211 | A1 | 12 September 2014 |
| US | 2019-0030089 | A1 | 31 January 2019 | AU | 2017-211244 | A1 | 09 August 2018 |
| | | | | AU | 2017-211244 | B2 | 16 May 2019 |
| | | | | BR | 112018015121 | A2 | 11 December 2018 |
| | | | | CA | 3012214 | A1 | 03 August 2017 |
| | | | | CN | 109310714 | A | 05 February 2019 |
| | | | | EP | 3407902 | A1 | 05 December 2018 |
| | | | | EP | 3407902 | B1 | 15 July 2020 |
| | | | | ES | 2820338 | T3 | 20 April 2021 |
| | | | | FR | 3046934 | A1 | 28 July 2017 |
| | | | | FR | 3046934 | B1 | 28 July 2017 |
| | | | | HU | E050810 | T2 | 28 January 2021 |
| | | | | IL | 260752 | A | 28 February 2021 |
| | | | | JP | 2019-508486 | A | 28 March 2019 |
| | | | | JP | 6935632 | B2 | 15 September 2021 |
| | | | | LT | 3407902 | T | 28 December 2020 |
| | | | | NZ | 744528 | A | 28 February 2020 |
| | | | | PL | 3407902 | T3 | 06 April 2021 |
| | | | | PT | 3407902 | T | 03 September 2020 |
| | | | | SG | 11201806361 | A | 30 August 2018 |
| | | | | US | 10918678 | B2 | 16 February 2021 |
| | | | | WO | 2017-129515 | A1 | 03 August 2017 |
| | | | | ZA | 201805302 | B | 30 October 2019 |
| US | 2017-0348360 | A1 | 07 December 2017 | AU | 2017-274416 | A1 | 03 January 2019 |
| | | | | CA | 3026414 | A1 | 07 December 2017 |
| | | | | CN | 109803534 | A | 24 May 2019 |
| | | | | EP | 3462882 | A1 | 10 April 2019 |
| | | | | EP | 3462882 | A4 | 22 January 2020 |
| | | | | JP | 2019-520340 | A | 18 July 2019 |
| | | | | WO | 2017-210428 | A1 | 07 December 2017 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200027900

**Non-patent literature cited in the description**

- **MILLER, L. T.** *J. Clin. Microbiol.,* 1982, vol. 18, 861-867
- **MINIKIN, D.E. et al.** *J Microbial Meth,* 1984, vol. 2, 233-241
- **TAKESHI FURUHASHI et al.** *Analytical Biochemistry,* 2018, vol. 543, 51-54